(19) Europäisches Patentamt
European Patent Office
Office européen des brevets

(11) Veröffentlichungsnummer: **0 187 329**
**B1**

(12) **EUROPÄISCHE PATENTSCHRIFT**

(45) Veröffentlichungstag der Patentschrift: **08.03.89**

(21) Anmeldenummer: **85116194.3**

(22) Anmeldetag: **18.12.85**

(51) Int. Cl.⁴: **B 41 M 5/12,** C 07 D 265/14, C 07 D 413/04, C 07 D 413/06, C 07 D 413/10, C 07 D 413/14, C 07 C 103/30

(54) **Chromogene 3,1-Benzoxazine.**

(30) Priorität: **08.01.85 DE 3500361**

(43) Veröffentlichungstag der Anmeldung: **16.07.86 Patentblatt 86/29**

(45) Bekanntmachung des Hinweises auf die Patenterteilung: **08.03.89 Patentblatt 89/10**

(84) Benannte Vertragsstaaten: **CH DE FR GB LI**

(56) Entgegenhaltungen:
**DE-A-2 518 096**
**DE-B-2 530 464**
**US-A-3 839 447**

**Chemical Abstracts, Band 68, Nr.5, 29. Jänner 1968, Columbus, Ohio, USA**
**Chemical Abstracts, Band 70, Nr. 11, 17. März 1969, Columbus, Ohio, USA**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Berneth, Horst, Dr., Erfurter Strasse 1, D-5090 Leverkusen (DE)**

**EP 0 187 329 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Gegenstand der Erfindung sind chromogene 3,1-Benzoxazine der Formel I

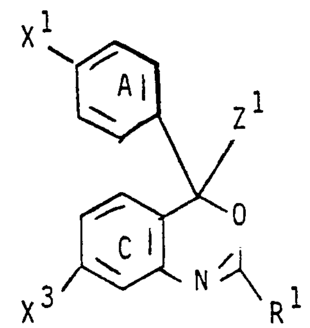

(I),

worin

$Z^1$ Wasserstoff, Alkyl, Cycloalkyl oder 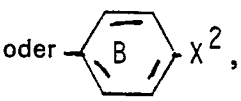,

$R^1$ Wasserstoff, Alkyl, Cycloalkyl, Aralkyl, Aryl oder gegebenenfalls über Methylen oder Ethylen gebundenes Heteryl,

$X^1$, $X^2$ und $X^3$ unabhängig voneinander Wasserstoff, Halogen, Alkyl, Cycloalkyl, Aryl, Alkanoylamino, Aroylamino, Heteryl, $NY^1Y^2$, $OY^3$ oder $SY^3$, wobei wenigstens einer der Reste $X^1$, $X^2$ oder $X^3$ für $NY^1Y^2$, $OY^3$ oder $SY^3$ steht,

$Y^1$, $Y^2$ und $Y^3$ unabhängig voneinander Wasserstoff, Alkyl, Cycloalkyl, Aralkyl, Aryl oder Heteryl oder die restlichen Glieder eines zu einem der o-ständigen Benzol-C-Atome reichenden, gegebenenfalls weitere Heteroatome enthaltenden 5- oder 6-gliedrigen Ringes, oder

$Y^1 + Y^2$ die restlichen Glieder eines gegebenenfalls weitere Heteroatome enthaltenden 5- oder 6-gliedrigen Ringes bedeuten,

die Ringe A, B und C sowie die genannten Reste ihrerseits in der Farbstoffchemie übliche nichtionische Reste tragen oder die Ringe A, B und C benzanelliert sein können, und worin, wenn die Ringe A und B keine weiteren Substituenten und Anellierungen tragen und

- wenn $X^3$ für Wasserstoff, Dialkylamino oder Diarylamino steht -
$X^1$ und $X^2$ nicht gleichzeitig Dimethylamino sind, oder

- wenn $R^1$ für 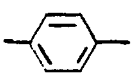 $OCH_3$ steht -
$X^1$ und $X^2$ nicht gleichzeitig Methoxy sind, oder

- wenn $Z^1$ für Wasserstoff steht -
$X^1$ nicht $C_1$- bis $C_3$-Alkoxy ist,

und ihre Verwendung für druckkopierfähige, thermoreaktive oder elektrochrome Aufzeichnungsmaterialien, die einen sauren Farbentwickler enthalten.

In der Farbstoffchemie übliche nichtionische Substituenten sind z. B.: Halogen, Hydroxy, Alkoxy, Aryloxy, Aralkoxy, Heteryloxy, Aryl, Heteryl, Alkylmercapto, Arylmercapto, Aralkylmercapto, Alkylsulfonyl, Cyan, Carbamoyl, Alkoxycarbonyl, Amino, das durch 1 oder 2 Alkyl-, Aryl- oder Aralkylgruppen substituiert sein kann, oder dessen Substituenten ringgeschlossen sein können, Alkenyloxy, Alkylcarbonyloxy und Arylcarbonyloxy und als Substituenten der Ringe außerdem Alkyl, Aralkyl, Nitro, Alkenyl oder Arylvinyl.

Bevorzugt stehen Alkyl für $C_1$-$C_{30}$-Alkyl, insbesondere für $C_1$-$C_{12}$-Alkyl und ganz besonders für $C_1$-$C_4$-Alkyl, und Alkenyl für $C_2$-$C_5$-Alkenyl.

Unter Halogen ist im besonderen Chlor und Brom zu verstehen.

Die Alkylreste und die Alkylreste in Alkoxy-, Alkylthio-, Dialkylamino-, Alkanoylamino-, Alkylsulfonyl- und Alkoxycarbonylgruppen können verzweigt und beispielsweise durch Fluor, Chlor, $C_1$- bis $C_4$-Alkoxy, Cyano oder $C_1$-$C_4$-Alkoxycarbonyl substituiert sein; besondere Beispiele sind Methyl, Ethyl, Propyl, 2-Propyl, 2,2-Dimethylpropyl, 2-Butyl, 1-Hexyl, 1-Octyl, 1-Dodecyl, 1-Tetradecyl, 1-Hexadecyl, 1-Octadecyl, 2-Bornylmethyl, 2-Chlorethyl, 2-Cyanoethyl, 2-Methoxyethyl, 2-Ethoxycarbonylethyl, Trifluormethyl.

Insbesondere werden unter Cycloalkyl Cyclohexyl, unter Aryl Phenyl und Naphthyl, unter Aralkyl Benzyl und Phenethyl, unter Heteryl Pyridyl, Pyrimidyl, Pyrazinyl, Triazinyl, Imidazolyl, Oxazolyl, Thiazolyl, Triazolyl, Thiadiazolyl oder Tetrazolyl, die benzanelliert sein können, sowie ihre teilhydrierten oder ganz hydrierten Derivate verstanden. Die Ringe können durch nichtionische Substituenten, insbesondere durch $C_1$-$C_4$-Alkyl, $C_1$-$C_4$-Alkoxy, Cyan, Nitro oder Halogen substituiert sein.

Die Phenyl- und Naphthylreste und die Reste in Benzyl- oder Benzoylaminogruppen können bis zu 3 gleiche oder verschiedene Reste tragen.

Besondere Beispiele für substituierte Phenylreste sind 2-, 3- oder 4-Tolyl, 2-, 3- oder 4-Anisyl, 2-, 3- oder 4-Chlorphenyl, 2-, 3- oder 4-Nitrophenyl, 2-, 3- oder 4-Cyanophenyl, 2-, 3- oder 4-Ethoxycarbonyl-phenyl, 2-, 3-oder 4-Methoxysulfonyl-phenyl, 2-, 3- oder 4-Trifluormethylphenyl, 2,3-Dinitrophenyl, 3,4-Dimethylphenyl, 2-Chlor-4-nitrophenyl, 3-Chlor-4-nitrophenyl, 5-Chlor-2-methyl-4-nitrophenyl, 4-Chlor-3-methylphenyl, 3-Chlor-4-methoxyphenyl, 3-Chlor-4-trifluormethylphenyl, 3,4-Dicyanophenyl, 2,5-Dichlor-4-cyanophenyl, 2-Methyl-1-naphthyl.

Die heterocyclischen Reste können bis zu 4 gleiche oder verschiedene Reste tragen. Besondere Beispiele für substituierte heterocyclische Reste sind 2-Methyl-4-pyridyl, 4-Nitro-2-pyridyl, 4-Phenyl-thiazol-2-yl, 5-Methylbenzoxazolyl, 5-tert.-Butyl-benzthiazolyl, 1,2-Dimethylindol-3- oder 5-yl, 2,2,6,6-Tetramethyl-piperidin-4-yl.

Bevorzugtes Alkanoyl sind Acetyl und Propionyl, und bevorzugtes Aroyl ist Benzoyl.

Die 3,1-Benzoxazine der Formel (I) sind normalerweise farblos oder höchstens schwach gefärbt.

Als saure Entwickler sind insbesondere Tone, saure Oxide und saure Salze sowie monomere oder polymere Phenole oder Carbonsäuren zu nennen.

Wenn die Farbbildner mit dem sauren Entwickler in Kontakt gebracht werden, ergeben sich intensive blaue, grünblaue, grüne, schwarze, violette oder rote Farbtöne, die ausgezeichnet sublimations- und lichtecht sind. Durch Mischungen untereinander lassen sich marineblaue, graue oder schwarze Färbungen erzielen.

Sie sind auch wertvoll im Gemisch mit einem oder mehreren anderen bekannten Farbbildnern, z. B. 3,3-Bis-(aminophenyl)-phthaliden, 3,3-Bis-(indolyl)-phthaliden, 3-Amino-fluoranen, Spirodipyranen, Chromenoindolen, Phenoxazinen, Phenothiazinen, Carbazolyl-methanen oder anderen Triarylmethanleukofarbstoffen, um grüne, violette, blaue, marineblaue, graue oder schwarze Färbungen zu ergeben.

Die 3,1-Benzoxazine der Formel (I) zeigen sowohl auf phenolischen Unterlagen wie auch besonders auf aktivierten Tonen eine gute Farbintensität. Sie eignen sich vor allem als Farbbildner für die Verwendung in einem wärmeempfindlichen oder druckempfindlichen Aufzeichnungsmaterial, das sowohl Kopier- als auch Registriermaterial sein kann. Ihre Entwicklungsgeschwindigkeit ist von den Substituenten nahezu unabhängig. Im allgemeinen zeichnen sie sich durch eine hohe Entwicklungsgeschwindigkeit aus bei gleichzeitig reduzierter Empfindlichkeit der Aufzeichnungsmaterialien gegenüber unbeabsichtigter vorzeitiger Entwicklung. Dadurch sind sie untereinander nahezu beliebig kombinierbar. Der Entwicklungsfarbton wird sofort erreicht, ohne daß unerwünschte Nuancenveränderungen während oder im Anschluß an die Entwicklung auftreten.

Die 3,1-Benzoxazine der Formel (I) zeichnen sich durch gute Lichtechtheit und Klimaalterungsstabilität sowohl im entwickelten als auch unentwickelten Zustand aus.

Ein druckempfindliches Material besteht beispielsweise aus mindestens 1 Paar von Blättern, die mindestens einen Farbbildner der Formel (I), gelöst oder dispergiert in einem nichtflüchtigen organischen Lösungsmittel, und einen sauren Entwickler enthalten.

Typische Beispiele für solche Entwickler sind Aktivtonsubstanzen, wie Attapulguston, Säureton, Bentonit, Montmorillonit, aktivierter Ton, z. B. säureaktiviertes Bentonit oder Montmorillonit, Zeolith, Halloysit, Siliciumdioxid, Aluminiumoxid, Aluminiumsulfat, Aluminiumphosphat, Zinkchlorid oder aktivertes Kaolin. Weitere Entwickler sind sauer reagierende organische Verbindungen, wie gegebenenfalls ringsubstituierte Phenole, Salicylsäure oder Salicylsäureester und deren Metallsalze, ferner besonders sauer reagierendes polymeres Material, z. B. ein phenolisches Polymer, ein Alkylphenolacetylenharz, ein Maleinsäurekolophoniumharz oder ein teilweise oder vollständig hydrolysiertes Polymerisat von Maleinsäureanhydrid mit Styrol, Ethylen oder Vinylmethylether oder Carboxypolymethylen. Es können auch Mischungen der genannten polymeren Verbindungen eingesetzt werden. Bevorzugte Entwickler sind Säureton, säureaktiviertes Bentonit oder Montmorillonit, Zinksalicylate oder die Kondensationsprodukte von p-substituierten Phenolen mit Formaldehyd. Die letzteren können auch Zink enthalten.

Die Entwickler können zusätzlich auch im Gemisch mit anderen an sich unreaktiven oder wenig reaktiven Pigmenten eingesetzt werden.

Der Farbbildner liefert an den Punkten, an denen er mit dem Entwickler in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Entwickler getrennt. Dies kann zweckmäßig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen. Verfahren zur Herstellung derartiger Mikrokapseln sind bekannt.

Als nichtflüchtige Lösungsmittel sind z. B. partiell hydriertes Terphenyl, alkylierte Naphthaline oder Dibutylphthalat geeignet.

Bevorzugt wird eine Anordnung, bei der der eingekapselte Farbbildner in Form einer Schicht auf der Rückseite eines Übertragungsblattes und der Elektronenakzeptor in Form einer Schicht auf der Vorderseite eines Empfangsblattes vorhanden sind.

Eine andere Anordnung der Bestandteile besteht darin, daß die den Farbbildner enthaltenden Mikrokapseln und der Entwickler in oder auf dem gleichen Blatt in Form einer oder mehrerer Einzelschichten oder in der Papierpulpe vorliegen.

Die Verbindungen der Formel (I) können vorzugsweise auch als Farbbildner in einem thermoreaktiven Aufzeichnungsmaterial verwendet werden. Dieses enthält in der Regel mindestens einen Schichtträger, einen Farbbildner, einen Entwickler und gegebenenfalls auch ein Bindemittel.

Thermoreaktive Aufzeichnungssysteme umfassen z. B. wärmeempfindliche Aufzeichnungs- und Kopiermaterialien und -papiere. Diese Systeme werden beispielsweise zum Aufzeichnen von Informationen, z. B. in elektronischen Rechnern, Ferndruckern, Fernschreibern oder in Aufzeichnungsgeräten und

Meßinstrumenten, wie z. B. Elektrokardiographen, verwendet. Die Bilderzeugung (Markierung) kann auch manuell mit einer erhitzten Feder erfolgen. Eine weitere Einrichtung der Erzeugung von Markierungen mittels Wärme sind Laserstrahlen.

Das thermoreaktive Aufzeichnungsmaterial kann so aufgebaut sein, daß der Farbbildner in einer Bindemittelschicht gelöst oder dispergiert ist und in einer zweiten Schicht der Entwickler in dem Bindemittel gelöst oder dispergiert ist. Eine andere Möglichkeit besteht darin, daß sowohl der Farbbildner als auch der Entwickler in einer Schicht dispergiert sind. Das Bindemittel wird in spezifischen Bereichen mittels Wärme erweicht und an diesen Stellen, an denen Wärme angewendet wird, kommt der Farbbildner mit dem Entwickler in Kontakt, und es entwickelt sich sofort die erwünschte Farbe.

Als Entwickler eignen sich die gleichen Substanzen wie sie in druckempfindlichen Papieren verwendet werden. Vorzugsweise verwendet werden hier Phenolharze oder phenolische Verbindungen, wie sie beispielsweise in der DE-PS-1 251 348 beschrieben sind, z. B. 4-tert.-Butyl-phenol, 4-Phenyl-phenol, 4-Hydroxy-diphenylether, α-Naphthol, β-Naphthol, 4-Hydroxy-benzoesäuremethylester, 4-Hydroxy-acetophenon, 2,2'-Dihydroxydiphenyl, 4,4'-Isopropylidendiphenol, 4,4'-Isopropyliden-bis-(2-methyl-phenol), 4,4'-Bis-(hydroxyphenyl)-valeriansäure, Hydrochinon, Pyrogallol, Fluoroglucin, p-, m- oder o-Hydroxy-benzoesäure, Gallussäure oder 1-Hydroxy-2-napthoesäure sowie Borsäure und Dicarbonsäuren, vorzugsweise aliphatische Dicarbonsäuren, z. B. Weinsäure, Oxalsäure, Maleinsäure, Citronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die 3,1-Benzoxazine und der Entwickler in Wasser schwerlöslich oder unlöslich sind.

Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so daß der Farbbildner mit dem Entwickler in Kontakt kommt und sich eine Farbe bilden kann. Wasserlösliche oder mindestens in Wasser quellbare Bindemittel sind z. B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, Gelatine und Stärke.

Die thermoreaktiven Schichten können weitere Zusätze enthalten: zur Verbesserung des Weißgrades, zur Erleichterung des Bedruckens der Papiere, zur Verhinderung des Festklebens der erhitzten Feder und zur Farbbildung nur innerhalb eines begrenzten Temperaturbereiches.

Ein weiteres geeignetes thermoreaktives Aufzeichnungssystem ist in DE-OS-3 337 296 beschrieben. Sauermodifizierte Polymerisate vorzugsweise des Acrylnitrils wirken da als Entwickler.

Die beschriebenen Verfahren und Zubereitungen sind beispielsweise aus den US-Patentschriften 2 948 753, 3 096 189 und 3 193 404 und aus den deutschen Offenlegungsschriften 2 555 080 und 2 700 937 bekannt.

Aus der DE-A-2 518 096 ist die Verwendung von 3,1-Benzoxazinen als Farbbildner in Aufzeichnungsmaterialien bekannt. Die bekannten Farbbildner unterscheiden sich von den erfindungsgemäßen 3,1-Benzoxazinen durch die Substituenten an den Ringen A, B und C. Sie entwickeln bereits bei Kontakt mit Cellulosefasern zum Farbstoff.

Bevorzugt sind chromogene 3,1-Benzoxazine der Formel II

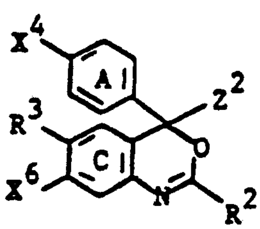

(II),

worin

einer der Reste

$X^6$ oder $R^3$ $NY^4Y^5$, $OY^6$ oder $SY^6$ und der andere Wasserstoff, $C_1$-$C_{18}$-Alkyl, das durch Fluor, Chlor, Brom, $C_1$-$C_8$-Alkoxy, Cyano und/oder $C_1$-$C_{18}$-Alkoxycarbonyl substituiert sein kann, Cyclohexyl, Benzyl-, Phenyl-, Biphenylyl- oder Naphthylreste, die durch Chlor, $C_1$-$C_8$-Alkyl, $C_1$-$C_8$-Alkoxy, Cyano, $C_1$-$C_4$-Alkoxycarbonyl und/oder $C_1$-$C_4$-Alkanoylamino substituiert sein können, oder $C_1$-$C_8$-Alkanoylamino oder Benzoylamino, das durch Chlor, $C_1$-$C_4$-Alkyl und/oder $C_1$-$C_4$-Alkoxy substituiert sein kann,

$Z^2$ Wasserstoff, $C_1$-$C_8$-Alkyl, Cyclohexyl oder 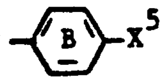,

$R^2$ Wasserstoff, gegebenenfalls Fluor, Chlor, Brom, $C_1$-$C_8$-Alkoxy, Cyano oder $C_1$-$C_{18}$-Alkoxycarbonyl tragendes $C_1$-$C_{18}$-Alkyl, Cyclohexyl, gegebenenfalls Fluor, Chlor, Brom, Nitro, $C_1$-$C_{18}$-Alkyl, $C_1$-$C_{18}$-Alkoxy, $C_1$-$C_{18}$-Alkylthio, $C_1$-$C_{18}$-Mono- oder Dialkylamino, $C_1$-$C_{18}$-Alkylsulfonyl, Cyano, $C_1$-$C_{18}$-Alkoxycarbonyl und/oder $C_1$-$C_{18}$-Alkanoylamino tragende Benzyl-, Phenyl-, Biphenylyl-, Terphenylyl-, Naphthyl-, Picolyl-, Pyridyl-, Chinolyl-, Pyrimidyl-, Pyrazinyl-, Triazinyl-, Triazolyl-, Triazolylmethyl-, Thiadiazolyl-, Tetrazolyl-, Indolyl-, gegebenenfalls benzanellierte Imidazol-, Oxazol- oder Thiazolreste,

$X^4$ und $X^5$ unabhängig voneinander Wasserstoff, Fluor, Chlor, Brom, $C_1$-$C_{18}$-Alkyl, gegebenenfalls durch Fluor, Chlor, Nitro, Hydroxy, $C_1$-$C_8$-Alkoxy, $C_1$-$C_8$-Alkylthio, Cyano, $C_1$-$C_8$-Alkoxycarbonyl, $C_1$-$C_8$-Alkanoyloxy, Amino und/oder Mono- oder Di-$C_1$-$C_4$-Alkylamino substituiertes $C_1$-$C_{18}$-Alkyl, gegebenenfalls durch Chlor und/oder $C_1$-$C_{12}$-Alkyl substituiertes Phenyl, $C_1$-$C_{12}$-Alkanoylamino, gegebenenfalls durch Chlor und/oder $C_1$-$C_{12}$-Alkyl substituiertes Benzoylamino, gegebenenfalls durch $C_1$-$C_4$-Alkyl, $C_1$-$C_4$-Alkoxy, Chlor und/oder Phenyl substituierte Indolyl- oder Piperidylreste, $NY^4Y^5$, $OY^6$ oder $SY^6$,

wobei aber höchstens zwei der Reste $X^4$, $X^5$ und $X^6$ $NY^4Y^5$ sind,

$Y^4$, $Y^5$ und $Y^6$ unabhängig voneinander Wasserstoff, gegebenenfalls durch Chlor, Hydroxy, Di-$C_1$-$C_4$-Alkylamino, Cyano, $C_1$-$C_4$-Alkoxycarbonyl, $C_1$-$C_4$-Alkanoyloxy oder $C_1$-$C_4$-Alkoxy substituiertes $C_1$-$C_{18}$-Alkyl, Cyclohexyl oder Phenyl oder Benzyl, die durch Chlor, $C_1$-$C_{12}$-Alkyl oder $C_1$-$C_{12}$-Alkoxy substituiert sein können, gegebenenfalls durch $C_1$-$C_4$-Alkyl substituiertes Piperidyl oder Glieder die zusammen mit N oder O, an die sie gebunden sind und einem der Ringe A, B oder C zur Vervollständigung eines Ringsystems der folgenden Formeln

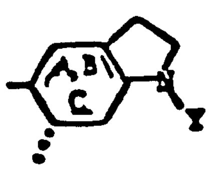 , 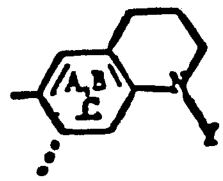 , 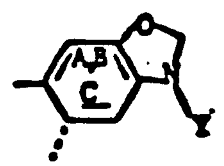 , 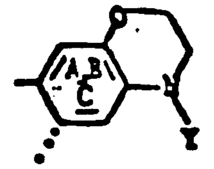 ,

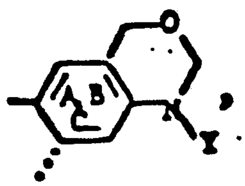 , 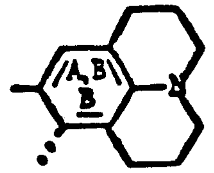 , 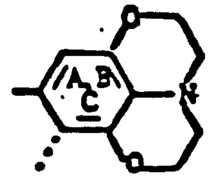 , 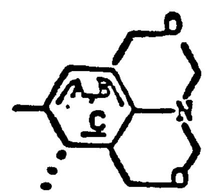 ,

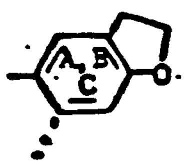 , 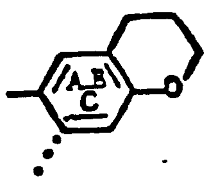 , 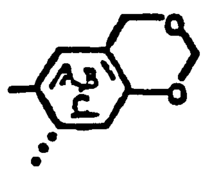 , 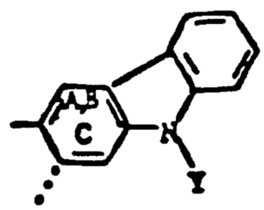

erforderlich sind, bedeuten, worin

die gestrichelte Linie die weitere Anellierung im Fall von Ring C bedeutet,

Y für Wasserstoff, $C_1$- bis $C_{18}$-Alkyl, das durch Chlor, Cyano, $C_1$- bis $C_4$-Alkoxycarbonyl oder $C_1$- bis $C_4$-Alkoxy substituiert sein kann, Cyclohexyl, Phenyl oder Benzyl, die durch Chlor, $C_1$- bis $C_{12}$-Alkyl oder $C_1$- bis $C_{12}$-Alkoxy substituiert sein können, steht,

der gesättigte Ringteil bis zu 4 Reste aus der Gruppe Chlor, $C_1$- bis $C_4$-Alkyl, $C_1$- bis $C_4$-Alkoxy oder Phenyl tragen kann,

die Ringe A, B und C durch Chlor, $C_1$-$C_4$-Alkyl, $C_1$-$C_4$-Alkoxy, gegebenenfalls durch Chlor, $C_1$-$C_4$-Alkyl oder $C_1$-$C_4$-Alkoxy substituiertes Phenoxy oder Phenylamino und/oder $C_1$-$C_4$-Alkanoylamino substituiert oder benzanelliert sein können, oder

$NY^4Y^5$ einen gegebenenfalls durch Chlor, $C_1$-$C_4$-Alkyl oder Aryl, insbesondere Phenyl substituierten Pyrrolo-, Pyrrolidino-, Piperidino-, Pipecolino-, Pyrazino-, Morpholino-, Pyrazolo- oder pyrazolinorest bedeuten.

Besonders bevorzugt sind chromogene 3,1-Benzoxazine der Formel III

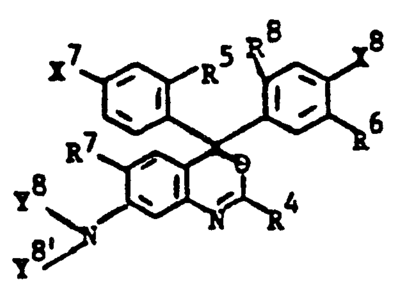 (III),

$X^8$ Wasserstoff, Chlor, Brom, gegebenenfalls durch Chlor und/oder $C_1$-$C_4$-Alkoxy substituiertes $C_1$-$C_{18}$-Alkyl, gegebenenfalls durch Chlor und/oder $C_1$-$C_4$-Alkyl substituiertes Phenyl, $C_1$-$C_4$-Alkanoylamino, gegebenenfalls durch Chlor und/oder $C_1$-$C_4$-Alkyl substituiertes Benzoylamino, gegebenenfalls durch Methyl, Ethyl, Methoxy,

Chlor und/oder Phenyl substituierte Indolyl- oder Piperidylreste, $OY^9$ oder $SY^9$,

$X^7$ $NY^7Y^{7}$ oder unabhängig von $X^8$ die bei $X^8$ angegebenen Reste,

$R^4$ Wasserstoff, gegebenenfalls Fluor, Chlor oder $C_1$-$C_4$-Alkoxy tragendes $C_1$-$C_{18}$-Alkyl, Cyclohexyl, gegebenenfalls Chlor und/oder $C_1$-$C_{18}$-Alkyl tragendes Benzyl, gegebenenfalls Chlor, Brom, Nitro, $C_1$-$C_8$-Alkyl, $C_1$-$C_8$-Alkoxy, $C_1$-$C_8$-Alkyithio, $C_1$-$C_8$-Dialkylamino, $C_1$-$C_8$-Alkylsulfonyl, Cyano, $C_1$-$C_8$-Alkoxycarbonyl und/oder $C_1$-$C_8$-Alkanoylamino tragende Phenyl-, Biphenylyl-, Naphthyl-, Picolyl-, Pyridyl-, Chinolyl-, Pyrimidyl-, Pyrazinyl-, Triazolyl-, Thiadiazolyl-, Indolyl-, gegebenenfalls benzanellierte Imidazol-, Oxazol- oder Thiazolreste,

$R^5$, $R^6$, $R^7$ und $R^8$ unabhängig voneinander Wasserstoff, Chlor, Brom, $C_1$-$C_4$-Alkyl, $C_1$-$C_4$-Alkoxy, $C_1$-$C_4$-Alkylthio, Amino, $C_1$-$C_4$-Mono- oder Dialkylamino, $C_1$-$C_4$-Alkanoylamino, $C_1$-$C_4$-Alkylsulfonylamino, gegebenenfalls durch Methyl, Methoxy, Ethoxy oder Chlor substituierte Benzoylamino-, Anilino- oder N-$C_1$-$C_4$-Alkylanilinoreste,

$Y^7$ bis $Y^9$ unabhängig voneinander gegebenenfalls durch Chlor, Cyano, Methoxycarbonyl, Methoxycarbonyloxy, Acetyloxy, Hydroxy, Methoxy, Ethoxy oder Dimethylamino substituiertes $C_1$-$C_8$-Alkyl, Cyclohexyl, Benzyl, Phenyl, das durch Chlor, Cyano, Methyl, Ethyl, Methoxy oder Ethoxy substituiert sein kann, 2,2,6,6-Tetramethyl- oder 1,2,2,6,6-Pentamethylpiperidin-4-yl bedeuten,

$Y^{7'}$ und $Y^{8'}$ Wasserstoff bedeuten oder die Bedeutung von $Y^7$ bzw. $Y^8$ besitzen, oder

$NY^7Y^{7'}$ und $NY^8Y^{8'}$ unabhängig voneinander für einen durch $C_1$-$C_4$-Alkyl und/oder Phenyl, das noch Chlor, Methyl, Methoxy, Ethoxy oder Cyano tragen kann substituierten Pyrrolidino-, Piperidino-, Piperazino-, Morpholino- oder Pyrazolinorest stehen.

In ganz besonders bevorzugten Verbindungen der Formel (III) stehen

$X^8$ für $X^{8'}$ = Wasserstoff, Chlor, $C_1$-$C_4$-Alkyl, $C_1$-$C_4$-Alkoxy, Benzyloxy, Phenoxy, Methylthio, Ethylthio, Phenyl, Tolyl, Chlorphenyl, Acetylamino, Propionylamino, Benzoylamino oder Chlorbenzoylamino,

$X^7$ für $X^{7'}$ = $NY^{7''}Y^{'''}$ oder unabhängig für die Bedeutung von $X^{8'}$,

$R^4$ für $R^{4'}$ = Wasserstoff, Methyl, Trifluormethyl, Ethyl, Propyl, Butyl, tert.-Butyl, Pentyl, Hexyl, Heptyl, Hept-3-yl, Octyl, Cyclohexyl, Benzyl, Phenyl, Chlorphenyl, Tolyl, tert.-Butylphenyl, Anisyl, Nitrophenyl, Cyanophenyl, Methoxycarbonylphenyl, Methylsulfonylphenyl, Chlornitrophenyl, Dichlorphenyl, Biphenylyl, Naphthyl, Pyridyl, Picolyl, Chinolyl, 2-, 3- oder 5-Indolyl oder 5-Benzoxazolyl,

$R^5$ für $R^{5'}$ und $R^8$ für $R^{8'}$ = Wasserstoff, Chlor, Methyl, Ethyl, Methoxy, Ethoxy, Amino, Methylamino, Acetylamino, Benzoylamino oder Methylsulfonylamino,

$R^6$ für $R^{6'}$ = Wasserstoff, Chlor, Brom, Methyl, Ethyl, Methoxy, Ethoxy, Acetylamino oder Methylsulfonylamino,

$R^7$ für $R^{7'}$ = Wasserstoff, Chlor, Brom, Methyl, Ethyl, Propyl, Butyl, Methoxy, Ethoxy, Acetylamino, Benzoylamino oder Methylsulfonylamino,

$Y^8$ für $Y^{8''}$ und $Y^{8'}$ für $Y^{8'''}$, wobei

$Y^{7''}$ und $Y^{8''}$ Methyl, Ethyl, Propyl, Butyl, Cyanethyl, Methoxyethyl, Ethoxyethyl, Methoxycarbonylethyl, Methoxycarbonyloxyethyl, Acetyloxyethyl, Benzyl, Phenyl, 4-Tolyl, 4-Chlorphenyl, 4-Anisyl, 4-Ethoxyphenyl oder 4-Cyanophenyl

$Y^{7'''}$ bzw. $Y^{8'''}$ Wasserstoff oder die Beteutung von $Y^{7''}$ bzw. $Y^{8''}$ oder

$NY^{7''}Y^{7'''}$ bzw. $NY^{8''}Y^{8'''}$

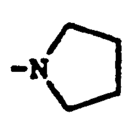 , 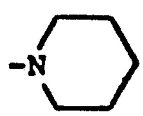 , 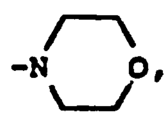 , 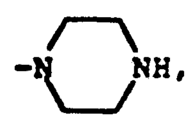 ,

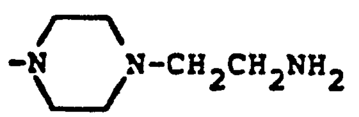 , 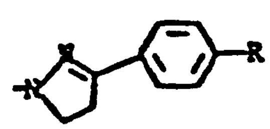

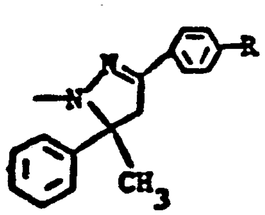 , 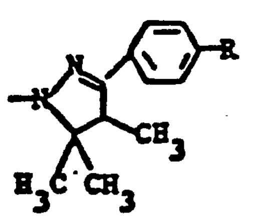 , 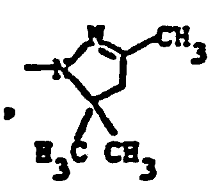

und

R Wasserstoff, Chlor, Methyl, Methoxy, Ethoxy oder Cyano bedeuten.

Ebenfalls besonders bevorzugt sind chromogene 3,1-Benzoxazine der Formel IV

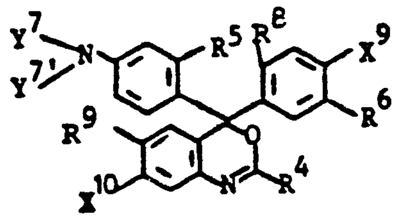

(IV),

worin

$X^9$ Wasserstoff, Chlor, Brom, gegebenenfalls durch Chlor und/oder $C_1$-$C_4$-Alkoxy substituiertes $C_1$-$C_{18}$-Alkyl, gegebenenfalls durch Chlor und/oder $C_1$-$C_4$-Alkyl substituiertes Phenyl, $C_1$-$C_4$-Alkanoylamino, gegebenenfalls durch Chlor und/oder $C_1$-$C_4$-Alkyl substituiertes Benzoylamino, gegebenenfalls durch Methyl, Ethyl, Methoxy, Chlor und/oder Phenyl substituierte Indolyl- oder Piperidylreste $NY^{10}Y^{10'}$, $OY^9$ oder $SY^9$,

$X^{10}$ $OY^{11}$ oder $SY^{11}$ und

$R^9$ Wasserstoff, Chlor, Brom, $C_1$-$C_4$-Alkyl, $C_1$-$C_4$-Alkoxy, $C_1$-$C_4$-Alkylthio, $C_1$-$C_4$-Alkanoylamino, Benzoylamino oder $C_1$-$C_4$-Alkylsulfonylamino oder

$X^{10}$ Wasserstoff, Chlor, Brom, gegebenenfalls durch Chlor und/oder $C_1$-$C_4$-Alkoxy substituiertes $C_1$-$C_{18}$-Alkyl, gegebenenfalls durch Chlor und/oder $C_1$-$C_4$-Alkyl substituiertes Phenyl, $C_1$-$C_4$-Alkanoylamino, gegebenenfalls durch Chlor und/oder $C_1$-$C_4$-Alkyl substituiertes Benzoylamino, gegebenenfalls durch Methyl, Ethyl, Methoxy, Chlor und/oder Phenyl substituierte Indolyl- oder Piperidylreste und

$C_1$-$C_4$-Alkoxy, $C_1$-$C_4$-Alkylthio, Amino, $C_1$-$C_4$-Mono- oder Dialkylamino, oder gegebenenfalls durch Methyl, Methoxy, Ethoxy oder Chlor substituiertes Anilino oder N-$C_1$-$C_4$-Alkylanilino bedeuten,

$R^4$, $R^5$, $R^6$ und $R^8$ die unter Formel (III) angegebene Bedeutung und

$Y^7$, $Y^{7'}$, $Y^9$ bis $Y^{11}$, $Y^{10'}$, $NY^7Y^{7'}$ und $NY^{10}Y^{10'}$ die unter Formel (III) für $Y^7$, $Y^{7'}$ bzw. $NY^7Y^{7'}$ angegebene Bedeutung besitzen.

In ganz besonders bevorzugten Verbindungen der Formel (IV) stehen

$X^9$ für $X^{9'}$ = Wasserstoff, Chlor, $C_1$-$C_4$-Alkyl, $C_1$-$C_4$-Alkoxy, Benzyloxy, Phenoxy, Methylthio, Ethylthio, Phenyl, Tolyl, Chlorphenyl, Acetylamino, Propionylamino, Benzoylamino, Chlorbenzoylamino oder $NY^{10''}Y^{10'''}$,

$X^{10}$ für $X^{10'}$ = $C_1$-$C_6$-Alkoxy, Benzyloxy, Phenoxy, Methylthio oder Ethylthio und

$R^9$ für $R^{9'}$ = Wasserstoff, Chlor, Methyl, Ethyl, Propyl, Butyl, Methoxy, Ethoxy, Acetylamino, Benzoylamino oder Methylsulfonylamino oder

$X^{10}$ für $X^{10'}$ = Wasserstoff, Chlor, $C_1$-$C_4$-Alkyl, Phenyl, Acetylamino, Propionylamino, Benzoylamino oder Chlorbenzoylamino und

$R^9$ für $R^{9'}$ = Methoxy, Ethoxy, Methylthio, Amino, Ethylamino, Dimethylamino, Anilino, N-Methyl-4-methylanilino oder N-Methyl-4-ethoxyanilino,

$R^4$, $R^5$, $R^6$ und $R^8$ für $R^{4'}$, $R^{5'}$, $R^{6'}$ bzw. $R^{8'}$ mit der unter Formel (III) angegebenen Bedeutung,

$Y^7$ für $Y^{7''}$ und $Y^{7'}$ für $Y^{7'''}$, wobei

$Y^{7''}$, $Y^{7'''}$, $Y^{10''}$, $Y^{10'''}$ und $NY^{7''}Y^{7'''}$ und $NY^{10''}Y^{10'''}$ die unter Formel (III) für $Y^{7''}$, $Y^{7'''}$ und $NY^{7''}Y^{7'''}$ angegebene Bedeutung besitzen.

Ebenfalls besonders bevorzugt sind chromogene 3,1-Benzoxazine der Formel V

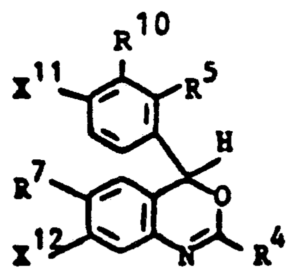

(V),

worin

$X^{11}$ und $X^{12}$ $NY^{12}Y^{12'}$, $OY^{13}$ oder $SY^{13}$ bedeuten und

$R^4$, $R^5$ und $R^7$ die bei Formel (III) angegebene Bedeutung besitzen,

$R^{10}$ die bei Formel (III) für $R^6$ angegebene Bedeutung und

$Y^{12}$, $Y^{12'}$, $Y^{13}$ und $NY^{12}Y^{12'}$ die bei Formel (III) für $Y^7$, $Y^{7'}$ bzw. $NY^7Y^{7'}$ angegebene Bedeutung besitzen.

In ganz besonders bevorzugten Verbindungen der Formel (V) stehen

$X^{11}$ und $C^{12}$ für $X^{11'}$ und $X^{12'}$ = $C_1$-$C_4$-Alkoxy, Benzyloxy, Phenoxy, Methylthio, Ethylthio oder $NY^{12''}Y^{12'''}$,

$R^4$, $R^5$ und $R^7$ für $R^{4'}$, $R^{5'}$ und $R^{7'}$ mit den unter Formel (III) angegebenen Bedeutungen,

$R^{10}$ für $R^{10'}$ mit der unter Formel (III) für $R^{6'}$ angegebenen Bedeutung und

$Y^{12''}$, $Y^{12'''}$ und $NY^{12''}Y^{12'''}$ besitzen die unter Formel (III) für $Y^{7''}$, $Y^{7'''}$ bzw. $NY^{7''}Y^{7'''}$ angegebene Bedeutung.

Ein weiterer Gegenstand der Erfindung sind Leukoverbindungen der Formel VI

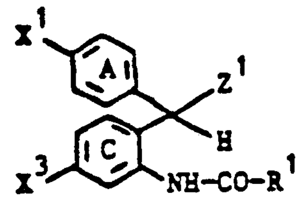

(VI),

worin
$R^1$, $Z^1$, $X^1$, $X^2$, $X^3$, A, B und C die oben angegebene Bedeutung besitzen, und wenn $Z^1$ für Wasserstoff steht und die Ringe A und C keine weiteren Substituenten oder anellierten Ringe tragen, $X^1$ und $X^3$ nicht gleichzeitig für 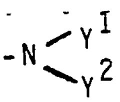 stehen.

Ein weiterer Gegenstand der Erfindung ist ein Verfahren zur Herstellung eines 3,1-Benzoxazins der Formeln (I) bis (V), dadurch gekennzeichnet, daß man ein Amid der Formel

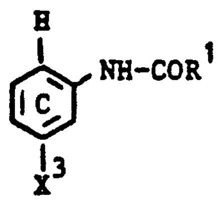

(VII),

mit einem Keton oder Aldehyd der Formel

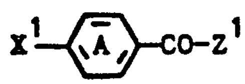

(VIII),

oder eine aromatische Verbindung der Formel

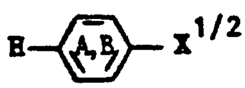

(IX),

mit einem Keton der Formel

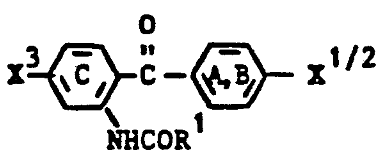

(X),

umsetzt, worin
$R^1$, $Z^1$, $X^1$, $X^2$, $X^3$, A, B und C die oben angegebene Bedeutung besitzen.
Insbesondere handelt es sich bei den Verbindungen (VII) und (IX) um solche, bei denen
$X^1$, $X^2$ oder $X^3$ ein Elektronendonorsubstituent ist, wie $NY^1Y^2$, $OY^3$ oder $SY^3$,
worin
$Y^1$ bis $Y^3$ die oben angegebene Bedeutung besitzen und die Ringe
A, B und C nicht durch starke Elektronenakzeptorsubstituenten, wie z. B. Nitro, Cyano oder Alkoxycarbonyl desaktiviert sind.

Die Umsetzung erfolgt üblicherweise mit wasserabspaltenden Reagenzien ohne oder auch mit unter diesen Bedingungen inerten Lösungsmitteln bei Temperaturen zwischen 0°C und dem Siedepunkt des jeweiligen Mediums. Anschließend wird evtl. nach Entfernen der inerten Lösungsmittel auf beispielsweise Wasser oder einen Alkohol ausgetragen. Durch Anheben des pH-Wertes mit beispielsweise Alkali- oder Erdalkalihydroxiden, Carbonaten, Hydrogencarbonaten, Ammoniak oder Aminen bis zum Verschwinden der Farbe dieser Mischung erhält man die 3,1-Benzoxazine der Formel (I). Hierbei kann es notwendig sein, zur Wasserabspaltung aus evtl. gebildeten Carbinolbasen einige Zeit zu erwärmen oder das primär erhaltene

unsaubere Produkt in Lösungsmitteln, wie Alkoholen - beispielsweise Methanol, Ethanol, 2-Propanol, Butanol; Nitrilen - beispielsweise Acetonitril; Ketonen - beispielsweise Aceton, 2-Butanon; (Chlor)Kohlenwasserstoffen - beispielsweise Toluol, Chlorbenzol, Dichlorbenzol, Chloroform, 1,2-Dichlorethan; oder Estern - beispielsweise Essigsäureethylester, Essigsäurebutylester, einige Zeit bei Temperaturen zwischen Raumtemperatur und der Siedetemperatur des jeweiligen Mediums zu behandeln.

Wasserabspaltende Reagenzien sind beispielsweise Phosphoroxychlorid, Phosphorpentachlorid, Diphosphorpentoxid, Triphenylphosphindichlorid, Phosgen, Phosphortrichlorid, Phosphortribromid, Sulfurylchlorid, Thionylchlorid, Oxalylchlorid oder Mischungen hiervon. Vorzugsweise werden Phosphoroxychlorid und Phosphoroxychlorid/Diphosphorpentoxid eingesetzt.

Geeignete inerte Lösungsmittel sind beispielsweise Toluol, Chlorbenzol, Dichlorbenzol, Nitrobenzol, chlorierte aliphatische Kohlenwasserstoffe, wie 1,2-Dichlorethan.

Die Herstellung der 3,1-Benzoxazine der Formel (I) kann auch durch Oxidation von Leukoverbindungen der Formel

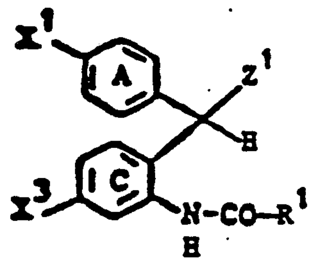

(VI)

worin

$R^1$, $Z^1$, $X^1$, $X^2$, $X^3$, A, B und C die oben angegebene Bedeutung besitzen, erfolgen.

Diese Oxidation kann auf bekannte Weise mit höherwertigen Metallverbindungen, wie $PbO_2$, $MnO_2$, Permanganaten, $CrO_3$, Chromaten, Dichromaten, $NiO_2$, $K_3$ $[Fe(CN)_6]$, mit Chinonen, wie Chloranil, Tetrachlor-o-chinon, Dichlordicyanochinon, oder auf eine andere literaturbekannte Weise, wie z. B. mit Sauerstoff, Luft, Perboraten oder Wasserstoffperoxid, erfolgen.

Die Aufarbeitung, Isolierung und evtl. Nachbehandlung erfolgt auf analoge Weise wie oben beschrieben.

Die Oxidation mit höherwertigen Metallverbindungen erfolgt üblicherweise in saurem Medium oder in organischen Lösungsmitteln, wie Alkoholen- z. B. Ethanol, Isopropanol, Ethylenglykolmonomethylether; Ketonen - z. B. Aceton, Butanon oder Methylisopropylketon; oder polaren aprotischen Lösungsmitteln, z. B. N-Methylpyrrolidon, γ-Butyrolacton, Acetonitril, Dimethylsulfoxid oder Sulfolan oder in Mischungen solcher Lösungsmittel mit Säuren bei Temperaturen zwischen 0° C und 60° C, vorzugsweise 10 - 40° C.

Geeignete Säuren sind beispielsweise Salzsäure, Schwefelsäure, Phosphorsäure, Essigsäure, Propionsäure oder Mischungen untereinander und/oder Mischungen mit Wasser. Eine bevorzugte Mischung ist Salzsäure, Essigsäure und Wasser.

Die Oxidation mit Chinonen erfolgt üblicherweise in organischen Lösungsmitteln, wie Alkoholen - z. B. Methanol, Ethanol, Isopropanol; Ketonen - z. B. Aceton, Butanon; Estern z. B. Essigsäureethyl- oder -butylester; Carbonsäuren - z. B. Essigsäure, Propionsäure; oder polaren aprotischen Lösungsmitteln, wie N-Methylpyrrolidon, Dimethylformamid, γ-Butyrolacton, Acetonitril, Sulfolan oder aber in Mischungen hiervon bei Temperaturen zwischen 0° C und dem Siedepunkt des Mediums, vorzugsweise 20 - 70° C.

Die Leukoverbindungen der Formel (VI) können beispielsweise als langsam entwickelnde Farbbildner in übliche druck- oder thermoreaktive Papiere eingearbeitet werden. Dort dienen sie wegen ihrer guten Lichtechtheit als Mischkomponenten für schnell entwickelnde aber wenig lichtechte Farbbildner, z. B. Kristallviolettlacton. So wird die Lichtechtheit der Aufzeichnung verbessert.

Außerdem sind die Leukoverbindungen der Formel (VI) geeignet für oxidativ-entwickelnde druck- oder thermoreaktive Aufzeichnungsmaterialien. Hier wird dem Farbbildner oder dem Entwickler ein geeignetes Oxidationsmittel zugesetzt, die weitere Verarbeitung der Schichten ist ähnlich wie bei üblichen thermo- oder druckempfindlichen Aufzeichnungsmaterialien. Durch Kontakt mit dem Oxidationsmittel wird die Leukoverbindung zum Farbstoff oxidiert und liefert so eine gefärbte Markierung.

Die 3,1-Benzoxazine der Formel (I) bzw. die hieraus durch Ringöffnung gebildeten Farbstoffe sind zum Anfärben von Polyacrylnitril, tannierter Baumwolle und anderen sauer modifizierten Fasern, Geweben und Pulvern geeignet.

**Beispiel 1**

270 g Michlers Hydrol und 137 g 3-Methoxy-4-methylanilin werden in 1 l Methanol und 5 ml konz. Salzsäure 2 h refluxiert. Nach dem Abkühlen wird abgesaugt, mit Methanol gewaschen und getrocknet. Umkristallisation aus Toluol ergibt 230 g (59 % der Theorie) farbloses Kristallisat vom Schmelzpunkt 190 bis 192°C und der

Formel

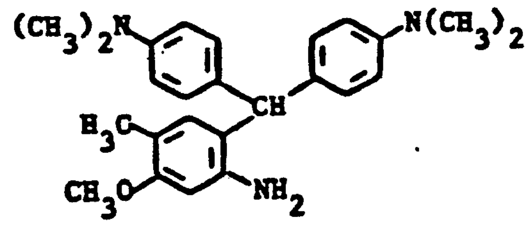

38,9 g dieser Verbindung werden in 100 ml 1,2-Dichlorethan suspendiert. 17,5 g 4-Chlor-benzoylchlorid in 50 ml 1,2-Dichlorethan werden dazugetropft. Nach 1 h bei 30 bis 40° C wird das Lösungsmittel im Vakuum entfernt, der Rückstand in 150 ml Ethanol aufgenommen und mit 10 %-iger Natronlauge neutralisiert. Die dicke Suspension wird abgesaugt, mit Ethanol/Wasser 3/1 gewaschen und getrocknet. Man erhält 50,9 g (96 % der Theorie) farbloses Kristallpulver vom Schmelzpunkt 229 bis 231° C und der Formel

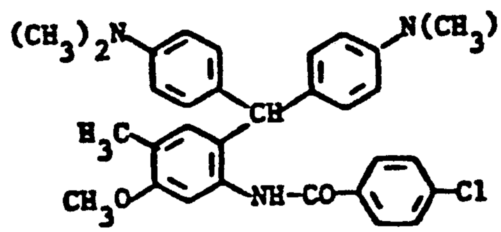

IR (KBr): 1673, 1610 $cm^{-1}$.

26,4 g dieser Leukoverbindung werden in 30 ml Dimethylformamid mit 12,3 g Chloranil bei 65° C 1,5 h gerührt. Nach dem Abkühlen wird mit 100 ml Methanol verdünnt, mit 17 ml Triethylamin entfärbt, abgesaugt und mit Methanol gewaschen. Noch feucht wird in 50 ml Acetonitril verrührt, abgesaugt und getrocknet:

22,8 g (87 % der Theorie) blaß grünliches Kristallisat vom Schmelzpunkt 237 bis 239° C und der Formel

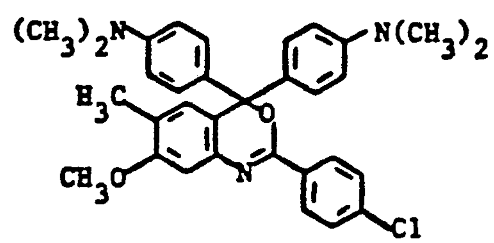

IR (KBr): 1610 $cm^{-1}$.

Eine Lösung in Eisessig wird blaustichig grün mit $\lambda_{max}$ = 475, 622 nm. Auf Säureton wird eine türkise, mit Bisphenol A eine blaustichig grüne intensive Färbung erzielt.

**Beispiel 2**

Analog Beispiel 1 erhält man die Verbindung der Formel

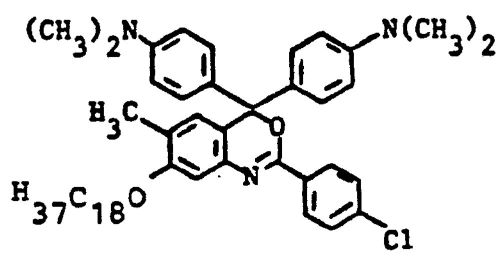

mit dem Schmelzpunkt 111 bis 113 C.

IR (KBr): 1610 $cm^{-1}$.

Eine Lösung in Eisessig wird blaustichig grün mit $\lambda_{max}$ = 475, 622 nm. Auf Säureton wird eine türkise, mit Bisphenol A eine blaustichig grüne, intensive Färbung erzielt.

Analog lassen sich die Verbindungen der Beispiele 3 bis 9 herstellen.

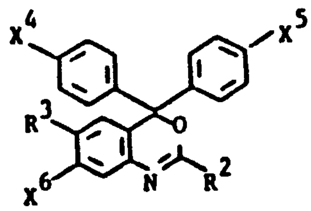

| Beisp. | $X^4$ | $X^5$ | $X^6$ | $R^3$ | $R^2$ | Farbton auf Säureton oder mit Bisphenol A |
|---|---|---|---|---|---|---|
| 3 | $(C_2H_5)_2N$ | $(C_2H_5)_2N$ | $CH_3O$ | $CH_3$ | 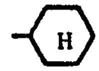 | türkis |
| 4 | $H_2N$ | $H_2N$ | 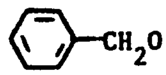 | $C_4H_9$ | 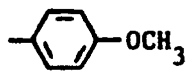 | " |
| 5 | $(CH_3)_2N$ | 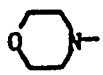 | $C_2H_5O$ | Cl | 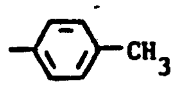 | blaustichig grün |
| 6 | $(CH_3)_2N$ | 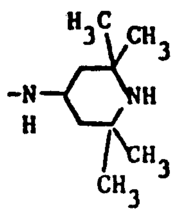 | $CH_3O$ | $CH_3$ | $-C_2H_5$ | " |
| 7 | $(C_2H_5)_2N$ | $(NCC_2H_4)_2N$ | H | $CH_3O$ | 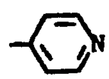 | |
| 8 | $(CH_3)_2N$ | 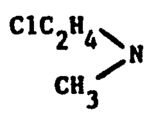 | 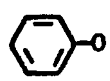 | $CH_3$ | 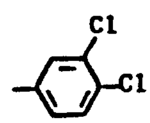 | " |
| 9 | " | $(CH_3)_2N$ | 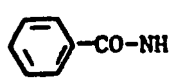 | H | 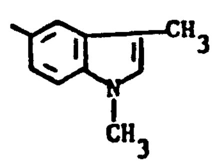 | " |

**Beispiel 10**

In eine Mischung aus 76,5 g Phosphoroxychlorid und 42,5 g Phosphorpentoxid wird eine Mischung aus 28,3 g 4-(Diethylamino)-4'-methoxybenzophenon und 27,5 g 3-(Dimethylamino)-4'-chlorbenzanilid unter Kühlung eingetragen. Die Schmelze wird 72 h bei 20 bis 25°C und 24 h bei 35 bis 40°C gerührt. Sie wird in 100 ml Acetonitril aufgenommen und auf eine Mischung aus 200 ml Chloroform und 500 g Eis bei 0°C ausgetragen. Bei 0 bis 5°C wird nun mit konz. Natronlauge auf pH = 11 gestellt und dieser pH beim Auftauen gehalten. Die Chloroformphase wird abgetrennt und über Natriumcarbonat getrocknet. Das Lösungsmittel wird im Vakuum entfernt, und der Rückstand wird in 100 ml Toluol gelöst und mit 500 ml 5 %-iger Salzsäure kräftig verrührt. Die Toluolphase wird abgetrennt und die wäßrige Phase noch dreimal mit je 100 ml Toluol extrahiert. Schließlich wird die saure wäßrige Phase in eine Lösung von 40 g Natriumhydroxid in 500 ml Eiswasser eingetropft. Die Fällung wird abgesaugt, mit Wasser gewaschen und noch feucht in 300 ml Ethanol eingetragen. Die Suspension wird auf 60°C aufgeheizt, abgekühlt und abgesaugt. Nach dem Trocknen wird erneut in 100 ml Aceton und 2 ml Triethylamin verrührt, abgesaugt und mit Aceton gewaschen. Nach dem Trocknen erhält man 19,4 g (36 % der Theorie) farbloses Kristallpulver vom Schmelzpunkt 164 bis 165°C und der Formel

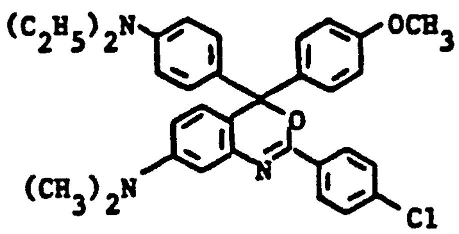

IR (KBr): 1600 $cm^{-1}$.

Eine Lösung in Eisessig wird tiefgrün mit $\lambda_{max}$ = 482, 629 nm. Auf Säureton und mit Bisphenol A wird eine intensive grüne Färbung erzielt.

**Beispiel 11**

Analog erhält man in 31 % Ausbeute die Verbindung der Formel

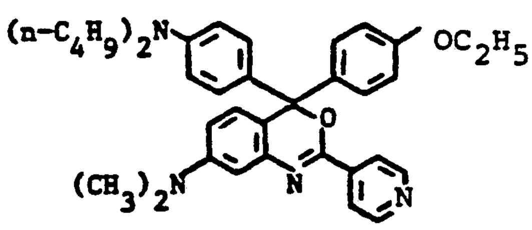

Eine Lösung in Eisessig wird tiefgrün mit $\lambda_{max}$ = 482, 630 nm. Auf Säureton und mit Bisphenol A wird eine intensive grüne Färbung erzielt.

**Beispiel 12**

Analog erhält man in 43 % Ausbeute die Verbindung der Formel

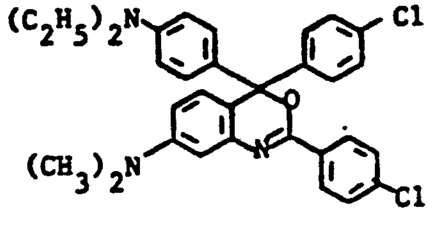

mit dem Schmelzpunkt 118 bis 120°C.

Eine Lösung in Eisessig wird tiefgrün mit $\lambda_{max}$ = 444, 648 nm. Auf Säureton und mit Bisphenol A wird eine intensive gelbstichig grüne Färbung erzielt.

**Beispiel 13**

Analog erhält man in 59 % Ausbeute die Verbindung der Formel

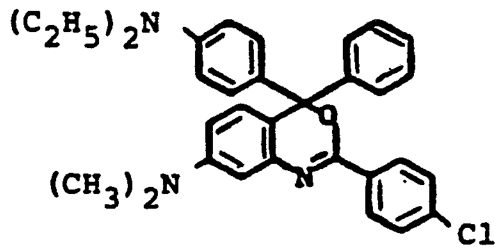

mit dem Schmelzpunkt 178 - 179°.
IR: 1598 $cm^{-1}$.
Eine Lösung in Eisessig wird tiefgrün mit $\lambda_{max}$ = 442, 645 nm. Auf Säureton und auf Bisphenol A wird eine intensive gelbstichig grüne Färbung erzielt.

**Beispiel 14**

Analog erhält man in 62 % Ausbeute die Verbindung der Formel

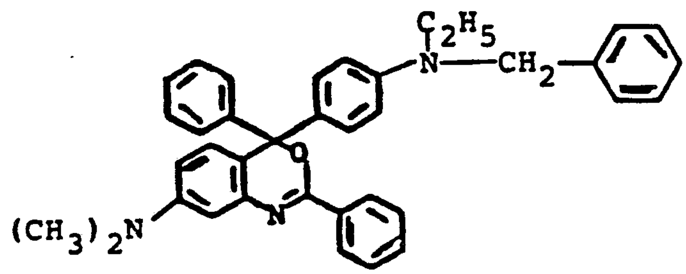

mit dem Schmelzpunkt 67 - 70°.
Eine Lösung in Eisessig wird grün mit $\lambda_{max}$ = 440, 648 nm. Auf Säureton oder mit Bisphenol A entwickelt sich eine intensive gelbstichig grüne Färbung.

**Beispiel 15**

Analog erhält man in 30 % Ausbeute die Verbindung der Formel

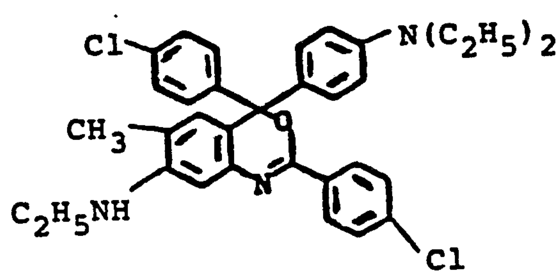

mit dem Schmelzpunkt 179° C.
Eine Lösung in Eisessig wird grün mit $\lambda_{max}$ = 442, 644 nm. Auf Säureton oder mit Bisphenol A entwickelt sich eine intensive gelbstichig grüne Färbung.

**Beispiel 16**

Analog erhält man in 35 % Ausbeute die Verbindung der Formel

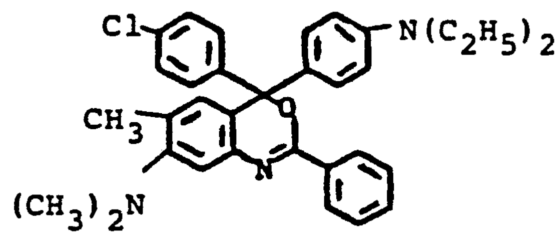

mit dem Schmelzpunkt 170 - 172°C.

Eine Lösung in Eisessig wird schwarzgurün mit $\lambda_{max}$ = 450, 632 nm.

Auf Säureton und mit Bisphenol A entwickelt sich eine grüne intensive Färbung.

**Beispiel 17**

Analog erhält man in 48 % Ausbeute die Verbindung der Formel

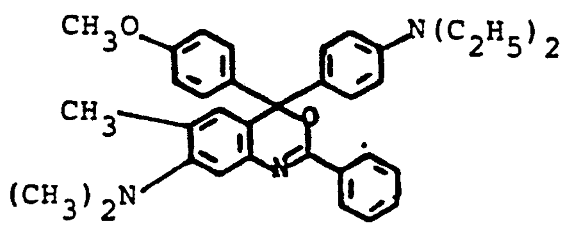

mit dem Schmelzpunkt 159 - 161°C.

Eine Lösung in Eisessig wird schwarz mit $\lambda_{max}$ = 491, 592 nm. Auf Säureton und mit Bisphenol A entwickelt sich eine intensive schwarze Färbung.

**Beispiel 18**

Analog erhält man in 45 % Ausbeute die Verbindung der Formel

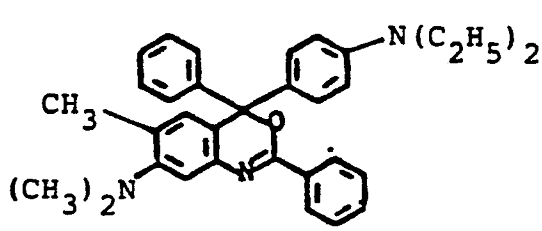

mit dem Schmelzpunkt 142 - 143°C.

Eine Lösung in Eisessig wird grün mit $\lambda_{max}$ = 448, 628 nm. Auf Säureton und mit Bisphenol A wird eine schmutzig grüne Färbung entwickeit.

Analog lassen sich die Verbindungen der Beispiele 19 bis 31 herstellen.

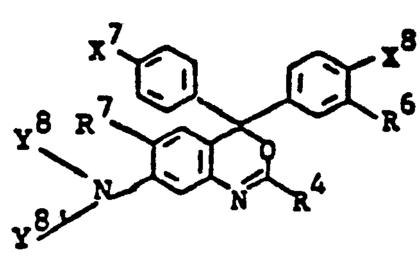

| Beisp. | $X^7$ | $X^8$ | $R^6$ | $Y^8$ | $Y^{8'}$ | $R^7$ | $R^4$ | Farbton auf Säureton oder mit Bisphenol A |
|---|---|---|---|---|---|---|---|---|
| 19 | $N(CH_3)_2$ | 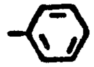 | H | $CH_3$ | $CH_3$ | H | H | grün |
| 20 | 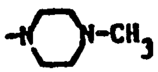 | $-SCH_3$ | H | $C_2H_5$ | H | $CH_3O$ | $C_2H_5$ | " |
| 21 | 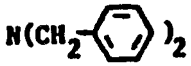 | 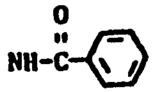 | H | $CH_3$ | $CH_3$ | H | 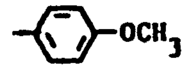 | " |
| 22 | $N(C_2H_4OCOCH_3)_2$ | Br | $CH_3$ | $C_2H_5$ | $C_2H_5$ | " | 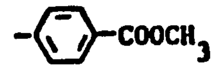 | gelbstichig grün |
| 23 | $N(C_2H_5)_2$ | $OCH_3$ | H | $CH_3$ | $CH_3$ | $CH_3$ | $-C(CH_3)_3$ | schwarz |
| 24 | 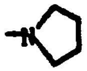 | 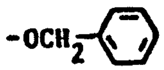 | $CH_3$ | $CH_3$ | $CH_3$ | H | 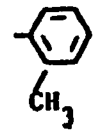 | grün |
| 25 | 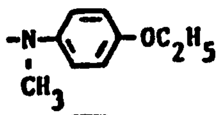 | Cl | $CH_3$ | $CH_3$ | $CH_2CH_2OCH_3$ | H | 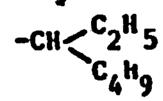 | grün |
| 26 | 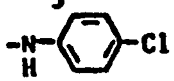 | $OC_2H_5$ | H | $CH_3$ | $C_{18}H_{37}$ | H | 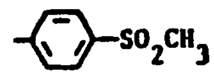 | " |
| 27 | 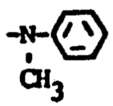 | 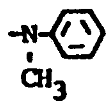 | H | $CH_3$ | $CH_3$ | H | 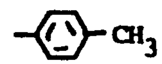 | blau |
| 28 | $-OCH_3$ | 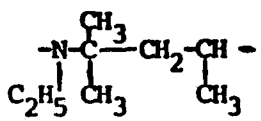 | | $CH_3$ | $CH_3$ | $CH_3$ | 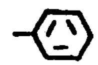 | blaustichig grün |
| 29 | $-N(C_2H_5)_2$ | H | $C_2H_5$ | $CH_3$ | $CH_3$ | $CH_3$ | 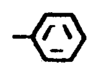 | stumpfes grün |
| 30 | $-N(C_2H_5)_2$ | $CH_3O$ | H | $C_2H_5$ | $C_2H_5$ | $CH_3$ | 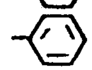 | schwarz |
| 31 | $-N(CH_3)_2$ | $CH_3O$ | $CH_3$ | $CH_3$ | $CH_3$ | H | 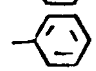 | stumpfes grün |

**Beispiel 32**

In 154,3 g Phosphoroxichlorid werden 72,2 g des Ketons der Formel

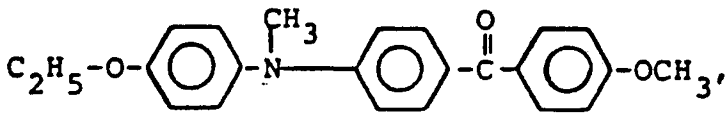

60,4 g des Amids der Formel

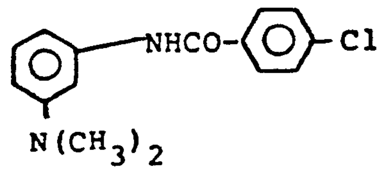

und 85,2 g Phosphorpentoxid eingetragen. Die Mischung wird 2 Tage bei 80°C gerührt und dann in 200 ml o-Dichlorbenzol aufgenommen. Bei Raumtemperatur wird auf 2 l Wasser ausgetragen und alkalisch gestellt. Die organische Phase wird abgetrennt und das Lösungsmittel mit Wasserdampf abdestilliert. Das beige Kristallisat wird abgesaugt und aus 2-Propanol umkristallisiert.

80,3 g (65 %) der Verbindung der Formel

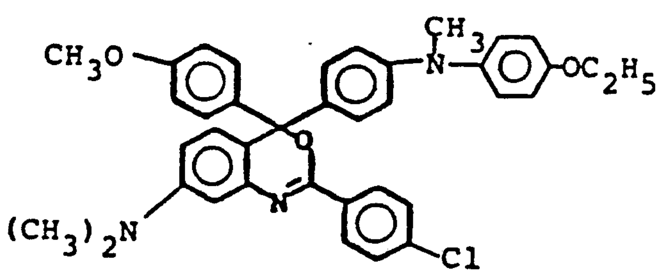

mit dem Schmelzpunkt 159 - 161°C.

IR (KBr): 1605 $cm^{-1}$

Eine Lösung in Eisessig wird grün mit $\lambda_{max}$ = 492, 636 nm. Auf Säureton und mit Bisphenol A wird eine intensive schmutzig grüne Färbung erzielt.

**Beispiel 33**

Analog erhält man in 45 % Ausbeute die Verbindung der Formel

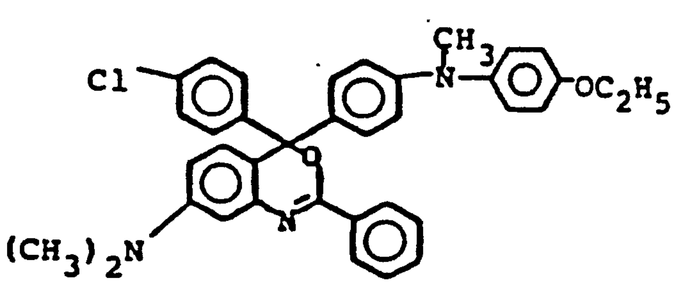

mit dem Schmelzpunkt 85 - 87°C.

Eine Lösung in Eisessig wird grün mit $\lambda_{max}$ = 455, 658 nm. Auf Säureton oder mit Bisphenol A wird eine intensive gelbstichig grüne Färbung erzielt.

Analog erhält man die Verbindungen der Beispiele 34 - 37.

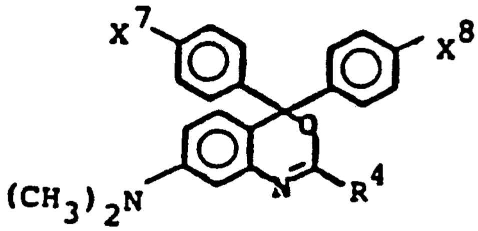

| Beisp. | $X_7$ | $X_8$ | $R^4$ | $\lambda_{max}$ in nm | Farbton auf Säureton oder mit Bisphenol A |
|---|---|---|---|---|---|
| 34 | $CH_3O$ | 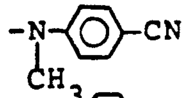 | 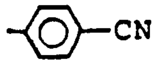 | 496,632nm | schwärzlich blau |
| 35 | H | 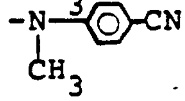 | 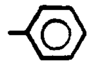 | 440,652nm | gelbstichig grün, stumpf |
| 36 | $CH_3O$ | 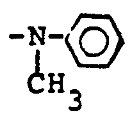 | 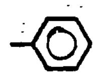 | 485,637nm | blaustichig grün, stumpf |
| 37 | Cl | 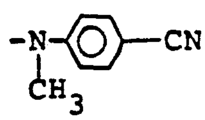 | 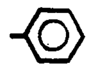 | 450,646nm | schmutzig grün |

**Beispiel 38**

In eine Mischung aus 38,3 g Phosphoroxychlorid und 28,3 g Phosphorpentoxid wird eine Mischung aus 14,4 g 4-Chlor-4'-(diethylamino)-benzophenon und 15,4 g 1-(3-Benzoylaminophenyl)-3,5,5-trimethyl-$\Delta^2$-pyrazolin unter Kühlung eingetragen. Anschließend wird 66 h bei 20 bis 25°C und 24 h bei 35 bis 40°C gerührt. Die grüne Schmelze wird in 50 ml Acetonitril gelöst und auf eine Mischung aus 200 ml Chloroform und 800 ml 10 %-iger Natronlauge ausgetragen. Die Chloroformphase wird abgetrennt, über Natriumcarbonat getrocknet und das Lösungsmittel entfernt. Das erhaltene braune Harz wird in 100 ml Methanol zur Kristallisation gebracht. Das gelbe Pulver wird abgesaugt und aus Toluol umkristallisiert. Man erhält 6,9 g (24 % der Theorie) hellgelbes Kristallisat vom Schmelzpunkt 235 bis 238° C und der Formel

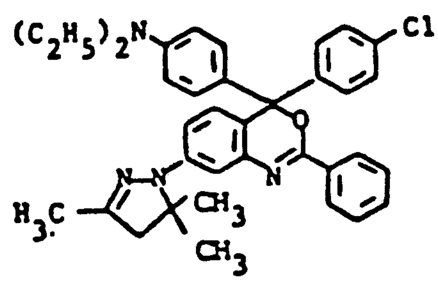

Eine Lösung in Eisessig wird tiefgrün mit $\lambda_{max}$ = 454, 674 nm. Auf Säureton und mit Bisphenol A wird eine intensive gelbstichig grüne Färbung erhalten.

**Beispiel 39**

37,7 g der analog Beispiel 1 hergestellten Leukoverbindung der Formel

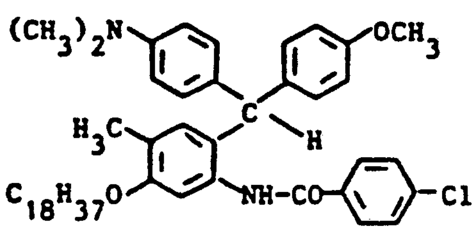

mit dem Schmelzpunkt 109 bis 111°C werden in 50 ml Dimethylformamid gelöst. Bei 60°C werden 12,3 g Chloranil zugegeben und die Mischung wird 1 h bei 65 bis 70°C gerührt. Dann wird abgekühlt, mit 100 ml Dimethylformamid verdünnt und mit ca. 10 ml 10 %-iger Natronlauge entfärbt. Nach 3 h Rühren wird von dem ausgefallenen Produkt abgesaugt und dieses mit 50 ml Ethanol kalt verrührt und dann nochmals mit 70 ml Ethanol und 1 ml Triethylamin aufgekocht. Schließlich wird abgesaugt, mit Ethanol gewaschen und im Vakuum getrocknet: 27,7 g (74 % der Theorie) blaßbeiges Kristallpulver vom Schmelzpunkt 94 bis 95°C und mit der Formel

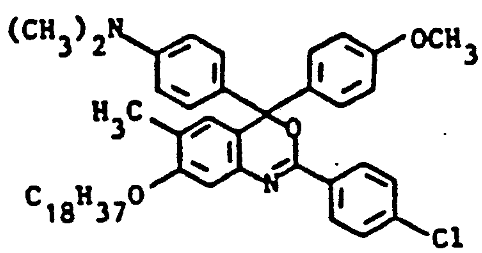

IR (KBr): 1605, 1595 $cm^{-1}$

Eine Lösung in Eisessig wird tiefbordo mit $\lambda_{max}$ = 548 nm. Auf Säureton und mit Bisphenol A wird ebenfalls eine tiefe Bordofärbung erzielt.

**Beispiel 40**

Analog läßt sich in 46 % Ausbeute die Verbindung der Formel

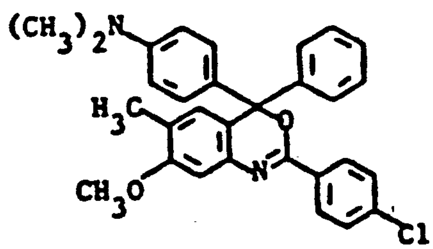

mit dem Schmelzpunkt 215 bis 217°C herstellen.

Eine Lösung in Eisessig wird tiefrot mit $\lambda_{max}$ = 511 nm. Auf Säureton und mit Bisphenol A wird eine intensive rote Färbung erzielt.

**Beispiel 41**

53,6 g Phosphoroxychlorid und 29,7 g Phosphorpentoxid werden im Eisbad vorgelegt. Hierzu wird eine Mischung aus 19,8 g 4-(Diethylamino)-4'-methoxybenzophenon und 19,4 g 3-Methoxy-4-methyl-4'-chlorbenzanilid eingetragen. Die bordofarbene Schmelze wird 88 h bei 25 bis 30°C gerührt und dann in 70 ml Acetonitril aufgenommen. Diese Lösung wird bei 10°C in 500 ml Wasser eingetropft und dann noch 1 h bei 20 bis 25°C verrührt. Vom abgeschiedenen Farbharz wird dekantiert und dieses in 300 ml Dimethylformamid gelöst. Diese Lösung wird nun in 1 l Wasser eingetropft, und anschließend wird mit 10 %-iger Natronlauge ein pH von 7 bis 8 eingestellt. Die Suspension wird auf 45°C erwärmt und der pH gehalten. Nach dem Abkühlen wird abgesaugt, mit Wasser gewaschen und im Vakuum getrocknet. Das gelbbeige Pulver wird nun

nacheinander mit 120 ml 2-Propanol, 50 ml 2-Propanol und 50 ml Methanol ausgekocht und jeweils nach dem Abkühlen abgesaugt und gewaschen und schließlich im Vakuum getrocknet: 24,7 g (65 % der Theorie) beiges Kristallpulver vom Schmelzpunkt 196 bis 199° C und mit der Formel

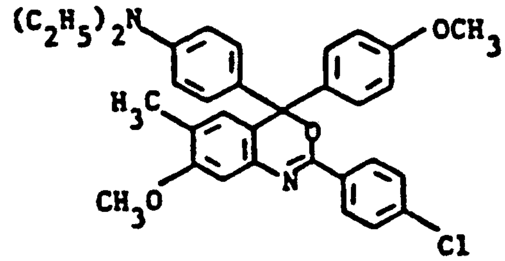

Eine Lösung in Eisessig wird tiefbordo mit $\lambda_{max}$ = 548 nm. Auf Säureton und mit Bisphenol A wird ebenfalls eine tiefe Bordofärbung erzielt.

Analog Beispiel 39 oder 41 werden die Verbindungen der Beispiele 42 bis 52 hergestellt.

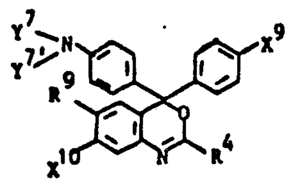

| Beisp. | $Y^7$ | $Y^{7'}$ | $X^9$ | $X^{10}$ | $R^9$ | $R^4$ | Schmp. | $\lambda$ max | Farbton auf Säureton und mit Bisphenol A |
|---|---|---|---|---|---|---|---|---|---|
| 42 | $C_2H_5$ | $C_2H_5$ | $CH_3O$ | $CH_3O$ | $CH_3$ | $CH_3$ | - | 540 nm | bordo |
| 43 | " | " | " | " | " | 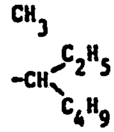 | - | 540 nm | " |
| 44 | " | " | " | " | " | $C_6H_5$ | 209°C | 548 nm | " |
| 45 | " | " | " | " | " | 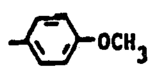 | 108°C | 549 nm | " |
| 46 | " | " | " | " | " | 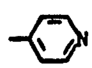 | - | 548 nm | " |
| 47 | " | " | " | " | $CH_3O$ | 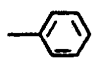 | 93-95°C | 554 nm | blaustichig rot |

| Beisp. | $Y^7$ | $Y^{7'}$ | $X^9$ | $X^{10}$ | $R^9$ | $R^4$ | Farbton auf Säureton und mit Bisphenol A |
|---|---|---|---|---|---|---|---|
| 48 | $C_6H_5$ | $CH_3$ | $C_2H_5O$ | $C_2H_5O$ | Cl | 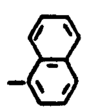 | rot |
| 49 | $CH_3$ | $CH_3$ | $(C_3H_7)_2N$ | H | 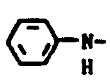 | 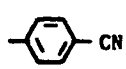 | grün |
| 50 | " | $C_2H_4CN$ | $C_6H_5CONH$ | $C_6H_5O$ | $CH_3O$ | $-C(CH_3)_3$ | " |
| 51 | $C_2H_5$ | $C_2H_5$ | Cl | $CH_3O$ | $CH_3$ | 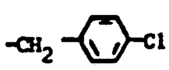 | rot |
| 52 | " | " | $CH_3O$ | $C_2H_5O$ | $CH_3$ | 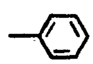 | bordo |

**Beispiel 53**

In eine Mischung aus 38,3 g Phosphoroxychlorid und 21,2 g Phosphorpentoxid wird unter Kühlung eine Mischung aus 12,8 g 4,4'-Dimethoxy-3-methylbenzophenon und 13,7 g 3-(Dimethylamino)-4'-chlorbenzanilid eingetragen. Die Schmelze wird 66 h bei 20 bis 25°C und 24 h bei 35 bis 40°C gerührt und dann in 50 ml Acetonitril aufgenommen. Diese Lösung tropft nun zu einer Mischung aus 200 ml Chloroform und 800 ml 10 %-iger Natronlauge. Die Chloroformphase wird abgetrennt, mit Natriumcarbonat getrocknet, über 200 g Kieselgel filtriert und das Lösungsmittel im Vakuum entfernt. Der Rückstand wird zweimal in 25 ml Methanol ausgekocht und getrocknet: 9,9 g (39 % der Theorie) hellgelbes Pulver vom Schmelzpunkt 112 bis 115°C und mit der Formel

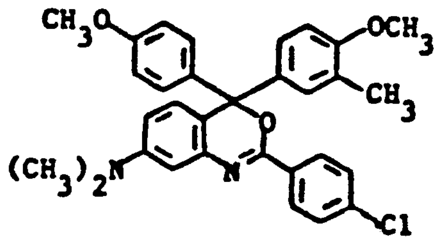

IR (KBr): 1598 $cm^{-1}$

Eine Lösung in Eisessig wird tiefviolett mit $\lambda_{max}$ = 456, 581 nm. Auf Säureton wird eine intensive schwärzlich violette, mit Bisphenol A eine graue Färbung erzielt.

**Beispiel 54**

Analog läßt sich in 88 % Ausbeute die Verbindung der Formel

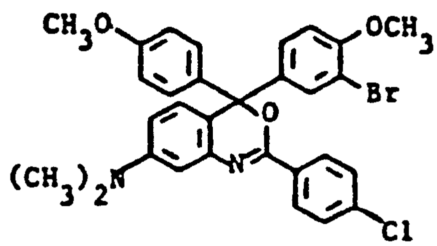

mit dem Schmelzpunkt 118 bis 122°C herstellen.

Eine Lösung in Eisessig wird violett mit $\lambda_{max}$ = 416, 571 nm. Auf Säureton wird eine violette Färbung erzielt.

**Beispiel 55**

Analog läßt sich in 56 % Ausbeute die Verbindung der Formel

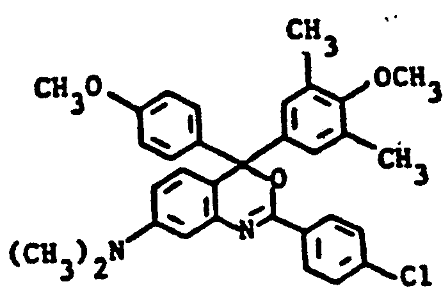

mit dem Schmelzpunkt 215 - 218°C herstellen.

Eine Lösung in Eisessig wird rotviolett mit $\lambda_{max}$ = 410, 566 nm. Auf Säureton und mit Bisphenol A wird eine rote bis violette Färbung erzielt.

**Beispiel 56**

Analog erhält man in 86 % Ausbeute die Verbindung der Formel

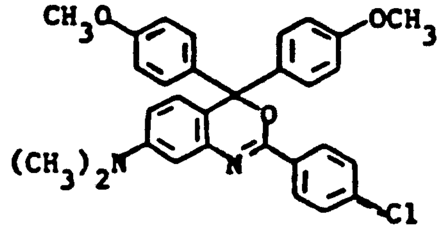

mit dem Schmelzpunkt 179 - 180°.

Eine Lösung in Eisessig wird tiefviolett mit $\lambda_{max}$ = 420, 446, 580 nm. Auf Säureton oder mit Bisphenol A erhält man eine violette bzw. graue Färbung.

Analog lassen sich auch die Beispiele 57 bis 66 herstellen:

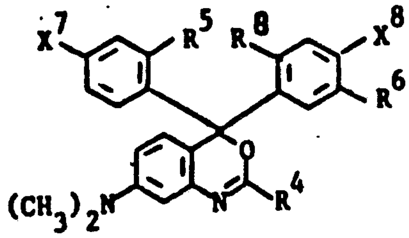

| Beisp. | $X^7$ | $X^8$ | $R^6$ | $R^5$ | $R^8$ | $R^4$ | Farbton auf Säureton oder mit Bisphenol A |
|---|---|---|---|---|---|---|---|
| 57 | H | $C_2H_5O$ | H | $CH_3O$ | H | 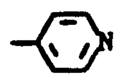 | rot |
| 58 | " | $CH_3$ | " | " | Cl | 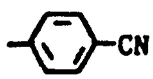 | " |
| 59 | $CH_3O$ | $CH_3O$ | $CH_3$ | H | " | 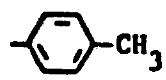 | rotviolett |
| 60 | $CH_3$ | " | Cl | " | H | 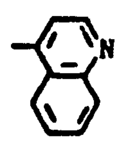 | orange |
| 61 | $C_2H_5O$ | $CH_3$ | $CH_3$ | " | $CH_3$ | 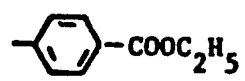 | orangerot |
| 62 | $CH_3O$ | $CH_3O$ | H | $CH_3O$ | H | 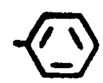 | rotviolett |
| 63 | $CH_3O$ | $CH_3O$ | $CH_3O$ | H | H | 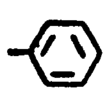 | " |
| 64 | $CH_3O$ | $N(CH_3)_2$ | H | H | $NHCH_3$ | 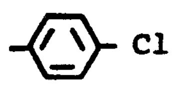 | schwarz |
| 65 | $CH_3O$ | $-N(CH_3)-C(CH_3)_2-CH_2-CH(CH_3)-$ | H | H | | 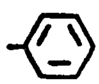 | blaustichig grün |
| 66 | $CH_3O$ | $CH_3O$ | $CH_3O$ | $CH_3O$ | H | $-C_3H_7$ | rotviolett |

**Beispiel 67**

In eine Mischung aus 38,3 g Phosphoroxychlorid und 21,2 g Phosphorpentoxid wird unter Kühlung eine Mischung aus 13,5 g 4,4'-Dimethoxy-3-methylbenzophenon und 14,0 g 3-Methoxy-4-methyl-4'-chlorbenzanilid eingetragen. Die rotorange Schmelze wird 48 h bei 20 bis 25°C und 24 h bei 35 bis 40°C gerührt und dann in 35 ml Acetonitril aufgenommen. Die Lösung tropft bei 0°C in 1 l Eiswasser. Diese Suspension wird ebenfalls bei 0°C mit konzentrierter Natronlauge alkalisch gestellt, auf Raumtemperatur kommen gelassen und alkalisch gehalten. Es wird abgesaugt, mit Wasser gewaschen und noch feucht in 150 ml Methanol verrührt. Nach dem Absaugen wird in 50 ml Methanol unter Zusatz von 10 ml Triethylamin 1/2 h refluxiert, abgekühlt, abgesaugt, mit Methanol gewaschen und im Vakuum getrocknet. Man erhält so 12,0 g (47 % der Theorie) farbloses Kristallpulver vom Schmelzpunkt 173 bis 177°C und der Formel

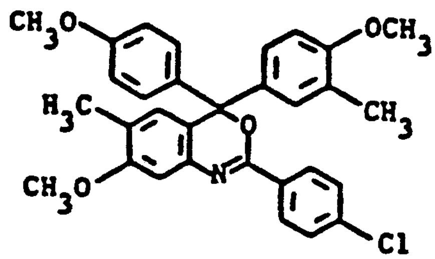

IR (KBr): 1595 $cm^{-1}$
Eine Lösung in Eisessig wird rot mit $\lambda_{max}$ = 485 nm. Auf Säureton wird eine rosa Färbung erzielt.

**Beispiel 68**

In eine Mischung aus 38,3 g Phosphoroxychlorid und 21,2 g Phosphorpentoxid wird unter Kühlung eine Mischung aus 14,9 g 4-(Diethylamino)-2-ethoxybenzophenon und 13,7 g 3-(Dimethylamino)-4'-chlorbenzanilid eingetragen. Die grüne Schmelze wird 136 h bei 20 bis 25°C gerührt und dann in 50 ml Acetonitril aufgenommen. Diese Lösung tropft bei 10°C in 300 ml Wasser. Nach 1 h bei 20 bis 25°C werden zu der braunen Lösung 45 g Natriumacetat zugesetzt und viermal mit 100 ml Toluol extrahiert. Schließlich wird mit konzentrierter Natronlauge auf pH = 7 gestellt, vom abgeschiedenen Farbharz dekantiert und dieses in 200 ml Dimethylformamid gelöst. Diese Lösung tropft in 1 l Wasser und wird mit 10 %-iger Natronlauge auf pH = 7 gestellt. Das ausflockende Produkt wird abgesaugt, mit Wasser gewaschen und im Vakuum getrocknet. Das gelbgrüne Pulver wird in 100 ml 2-Propanol 1 h verrührt, aufgekocht, nach dem Abkühlen abgesaugt, mit 2-Propanol gewaschen und im Vakuum getrocknet: 13,8 g (50 % der Theorie) beiges Kristallisat vom Schmelzpunkt 154 bis 157°C und mit der Formel

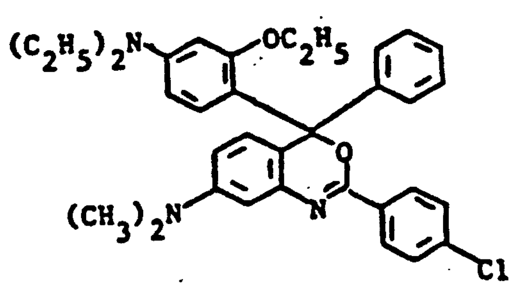

Eine Lösung in Eisessig wird gelbstichig grün mit $\lambda_{max}$ = 443, 656 nm. Auf Säureton und mit Bisphenol A wird eine intensive gelbstichig grüne Färbung erzielt.

**Beispiel 69**

Analog erhält man in 58 % Ausbeute die Verbindung der Formel

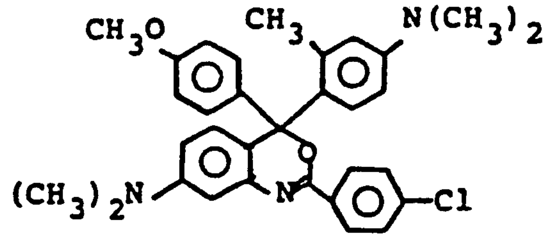

und dem Schmelzpunkt 230 - 233° C.
Eine Lösung in Eisessig wird grün mit $\lambda_{max}$ = 490, 643 nm.
Auf Säureton und auf Bisphenol A wird eine intensive graugrüne Färbung erzielt.

**Beispiel 70**

Analog läßt sich in 31 % Ausbeute die Verbindung der Formel

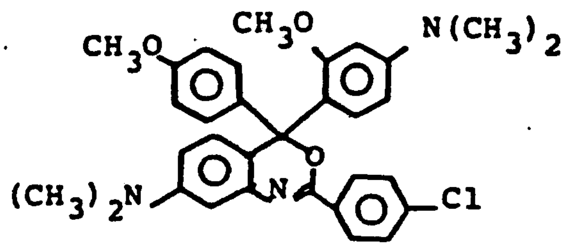

mit dem Schmelzpunkt 182° C herstellen.
Eine Lösung in Eisessig wird grün mit $\lambda_{max}$ = 478, 642 nm. Auf Säureton und mit Bisphenol A wird eine intensive gelbstichig graugrüne Färbung erzielt.

**Beispiel 71**

Analog wird in 46 % Ausbeute die Verbindung der Formel

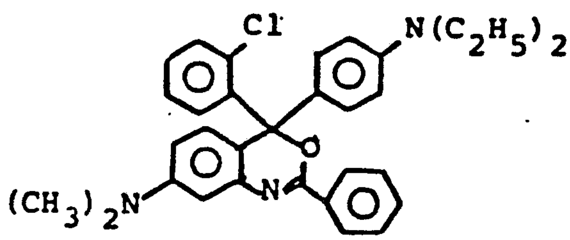

mit dem Schmelzpunkt 141 - 142° C (Zers.) erhalten.
Eine Lösung in Eisessig wird grün mit $\lambda_{max}$ = 426, 662 nm.
IR: 1608 $cm^{-1}$.
Auf Säureton und mit Bisphenol A wird eine intensive leuchtend, grüne Färbung erzielt.

**Beispiel 72**

13,6 g 4-Methoxy-benzaldehyd und 54,8 g 3-(Dimethylamino)-4'-chlorbenzanilid werden in 150 ml Ethanol mit 15 ml konzentrierter Salzsäure 3 h refluxiert. Nach dem Abkühlen wird mit 30 %-iger methanolischer Natriummethylatlösung alkalisch gestellt, filtriert und das Filtrat in 1,5 l Eiswasser eingetropft. Die Suspension wird abgesaugt, mit Aceton gewaschen und dann noch feucht in 100 ml Aceton 30 min refluxiert, abgekühlt, abgesaugt, mit Aceton gewaschen und im Vakuum getrocknet. Man erhält 45,4 g (68 % der Theorie) farbloses Kristallisat vom Schmelzpunkt 220 bis 222° C und der Formel

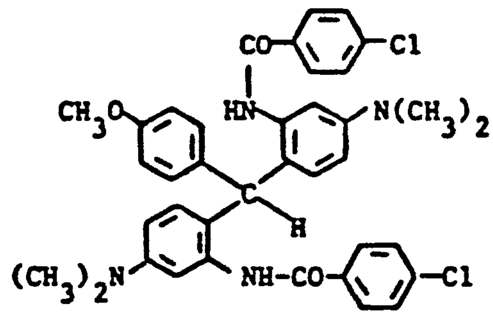

20 g dieser Leukoverbindung werden in 30 ml Dimethylformamid bei 60°C mit 8,2 g Chloranil versetzt und 1 h bei 65 bis 70°C gerührt. Nach dem Abkühlen wird mit 30 ml Methanol verdünnt und in 500 ml Wasser eingetropft. Mit 10 %-iger Natronlauge wird entfärbt und abgesaugt. Noch feucht wird in Methanol aufgekocht, mit 10 %-iger Natronlauge ein pH von 9 eingestellt, kalt gerührt, abgesaugt und mit Methanol/Wasser 1/1 gewaschen. Das Produkt wird im Vakuum getrocknet: 18,3 g (92 % der Theorie) hellgraues Kristallpulver vom Schmelzpunkt 189 bis 192°C und mit der Formel

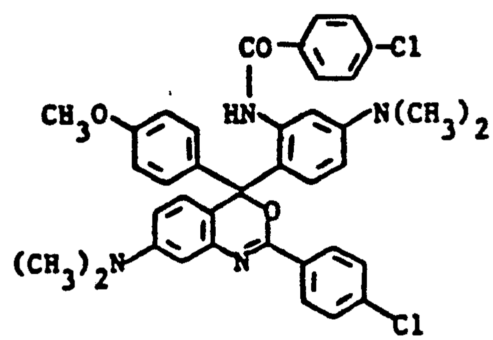

Eine Lösung in Eisessig wird schwarz mit $\lambda_{max}$ = 508, 660 nm. Auf Säureton und mit Bisphenöl A wird ebenfalls ein schwarzer Farbton entwickelt.

**Beispiel 73**

Analog läßt sich in 78 % Ausbeute die Verbindung der Formel

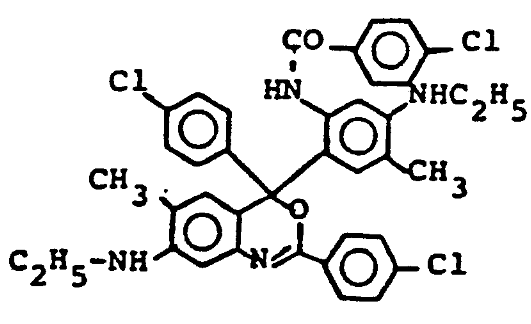

mit dem Schmelzpunkt 180 - 185°C herstellen.

Eine Lösung in Eisessig wird grün mit $\lambda_{max}$ = 452, 672 nm.

Auf Säureton und mit Bisphenol A wird eine schmutzig grüne Färbung erzielt.

**Beispiel 74**

Analog erhält man in 22 % Ausbeute die Verbindung der Formel

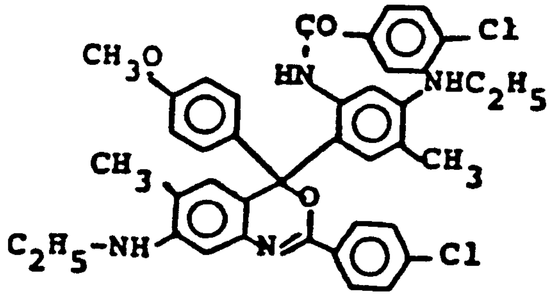

mit dem Schmeizpunkt 167 - 170° C.

Eine Lösung in Eisessig wird schwarz mit $\lambda_{max}$ = 501, 648 nm.

Auf Säureton und auf Bisphenol A wird ebenfalls eine intensive schwarze Färbung erzielt.

Analog lassen sich die Beispiele 75 - 82 herstellen.

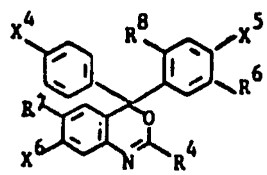

| Beisp. | $X^4$ | $X^5$ | $X^6$ | $R^6$ | $R^7$ | $R^8$ | $R^4$ | Farbton auf Säureton oder mit Bisphenol A |
|---|---|---|---|---|---|---|---|---|
| 75 | $OC_2H_5$ | $-N(CH_3)_2$ | $-N(CH_3)_2$ | H | H | $NHCOR^4$ | 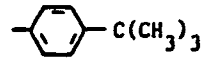 | schwarz |
| 76 | Cl | $-N(C_2H_5)_2$ | $-N(C_2H_5)_2$ | " | " | " | 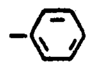 | grün |
| 77 | $-N(CH_3)_2$ | $-OCH_3$ | $-OCH_3$ | $CH_3$ | $CH_3$ | " | $-C_2H_5$ | rot |
| 78 | $CH_3$ | $-N(CH_3)_2$ | $-N(CH_3)_2$ | H | H | " | 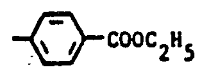 | grün |
| 79 | $OCH_3$ | $N(C_2H_5)_2$ | $N(CH_3)_2$ | H | H | $NHSO_2CH_3$ | 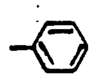 | schwarz |
| 80 | Cl | $N(C_3H_7)_2$ | $N(CH_3)_2$ | H | H | $NHSO_2CH_3$ | 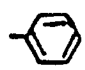 | grün |
| 81 | H | $N(CH_3)_2$ | $N(CH_3)_2$ | H | H | 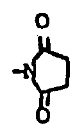 | 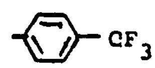 | grün |
| 82 | $OCH_3$ | $N(CH_3)_2$ | $N(CH_3)_2$ | $CH_3$ | $CH_3$ | $NHCOR^4$ | 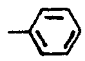 | schwarz |

**Beispiel 83**

28 g des analog A. Kliegl, Ber. Dtschen Chem. Ges. 39, 1266 (1906) erhaltenen Ketons der Formel

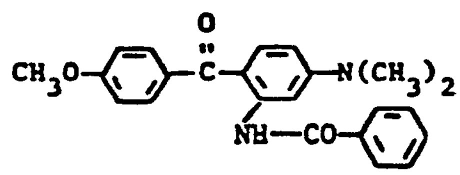

mit dem Schmelzpunkt 178 bis 179°C werden in 400 ml Ethanol mit 8,06 g Natriumboranat versetzt und 24 h bei 40°C in Suspension verrührt. Dann wird abgekühlt, mit 57 ml Aceton versetzt und nach 1 h Nachrühren abgesaugt und im Vakuum getrocknet. Dann wird in Toluol unter Zusatz katalytischer Mengen Essigsäure wasserfrei destilliert und das Lösungsmittel abgezogen. Man erhält 23 g (86 % der Theorie) blaß gelbliches Kristallisat vom Schmelzpunkt 115 - 118°C mit der Formel

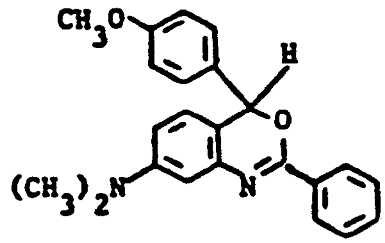

Eine Lösung in Eisessig wird orangerot mit $\lambda_{max}$ = 484 nm. Auf Säureton wird eine orange Färbung erzielt.

**Beispiel 84**

7,4 g der nach DE-PS-79 250 hergestellten Leukoverbindung der Formel

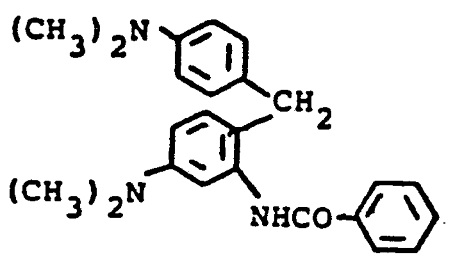

werden in 60 ml 80 proz. Essigsäure und 9 g konz. Salzsäure mit 15,4 ml einer 31 proz. wäßrigen Bleidioxidsuspension 30 min bei 30 - 35°C verrührt. Dann werden 14 ml 20 proz. Schwefelsäure zugesetzt, filtriert und auf pH = 1,9 gestellt. Die Fällung wird abgesaugt und verworfen. Dann wird auf pH = 3,4 gestellt und abgesaugt. Das Produkt wird in Ethanol aufgekocht und abgesaugt:

3,1 g (42 %) beiges Kristallisat der Formel

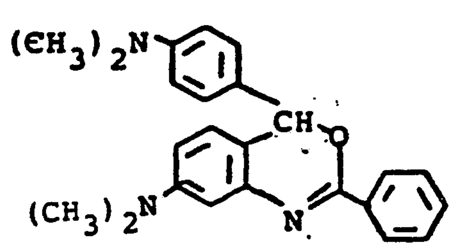

Eine Lösung in Eisessig wird blau mit $\lambda_{max}$ = 605 nm.

Auf Säureton und mit Bisphenol A wird ebenfalls eine intensive blaue Färbung erzielt.

Analog erhält man die Verbindungen der Beispiele 85 - 90.

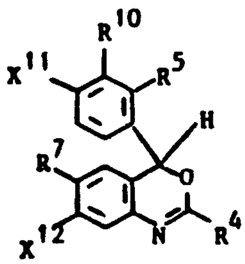

| Beisp. | $X^{11}$ | $X^{12}$ | $R^5$ | $R^7$ | $R^{10}$ | $R^4$ | Farbton auf Säureton |
|---|---|---|---|---|---|---|---|
| 85 | $C_2H_5O$ | $N(CH_3)_2$ | Cl | H | H | 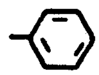 | orangerot |
| 86 | $N(C_2H_5)_2$ | $CH_3O$ | H | $CH_3$ | " | $CH_3$ | " |
| 87 | 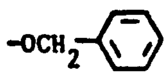 | 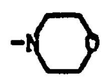 | " | H | $CH_3$ | 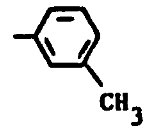 | " |
| 88 | $N(CH_3)_2$ | $N(CH_3)_2$ | " | $CH_3$ | H | 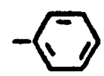 | blau |
| 89 | 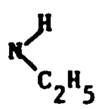 | 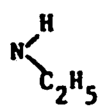 | " | $CH_3$ | $CH_3$ | 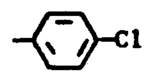 | " |
| 90 | $N(CH_3)_2$ | $N(CH_3)_2$ | 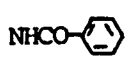 | H | H | 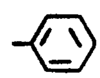 | türkis |

**Beispiel 91**

**Herstellung eines druckempfindlichen Kopierpapiers**

Eine Lösung von 3 g der 3,1-Benzoxazin-Verbindung des Beispiels 1 in 80 g Diisopropylnaphthalin und 17 g Kerosin wird auf an sich bekannte Weise mit Gelatine und Gummiarabicum durch Koazervation mikroverkapselt, mit Stärkelösung vermischt und auf ein Blatt Papier gestrichen. Ein zweites Blatt Papier wird auf der Frontseite mit säureaktiviertem Bentonit als Farbentwickler beschichtet. Das erste Blatt und das mit Farbentwickler beschichtete Blatt werden mit den Beschichtungen benachbart aufeinandergelegt. Durch Schreiben mit der Hand oder mit der Schreibmaschine auf dem ersten Blatt wird Druck ausgeübt, und es entwickelt sich auf dem mit dem Entwickler beschichteten Blatt eine intensive türkise Kopie, die ausgezeichnet lichtecht ist.

**Beispiel 92**

1 g der 3,1-Benzoxazin-Verbindung des Beispiels 72 wird in 17 g Toluol gelöst. Zu dieser Lösung gibt man unter Rühren 12 g Polyvinylacetat, 8 g Calciumcarbonat und 2 g Titandioxid. Die erhaltene Suspension wird im Gewichtsverhältnis 1/1 mit Toluol verdünnt und mit einem 10 µm Rakel auf ein Blatt Papier gestrichen. Auf dieses Blatt papier wird ein zweites Blatt Papier gelegt, dessen Unterseite bei einem Auftragsgewicht von 3 g/m² mit einer Mischung - bestehend aus 1 Teil Zinkchloridbeschichtet ist. Durch Schreiben mit der Hand oder mit der Schreibmaschine auf dem oberen Blatt wird Druck ausgeübt, und es entwickelt sich auf dem mit dem Farbbildner beschichteten Blatt eine intensive und lichtechte schwarze Farbe.

**Beispiel 93**

Man verfährt wie in Beispiel 91, nur setzt man eine Mischung aus 1,5 g der Verbindung des Beispiels 68 und 1,5 g der Verbindung des Beispiels 41 ein. So erhält man ein beschichtetes Blatt, das eine schwarze Kopie liefert, die ausgezeichnet lichtecht ist.

Analog werden die Mischungen der Beispiele 94 bis 104 hergestellt:

| Beispiel | Verbindung des Beispiels | Menge | Verbindung des Beispiels | Menge | Farbton |
|---|---|---|---|---|---|
| 94 | 12 | 1,8 g | 39 | 1,2 g | schwarz |
| 95 | 10 | 1,0 g | 47 | 2,0 g | schwarz |
| 96 | 10 | 1,5 g | 40 | 1,5 g | schwarz |
| 97 | 12 | 1,5 g | 40 | 1,5 g | schwarz |
| 98 | 35 | 1,5 g | 41 | 1,5 g | schwarz |
| 99 | 32 | 1,0 g | 53 | 2,0 g | schwarz |
| 100 | 71 | 1,2 g | 41 | 1,8 g | schwarz |
| 101 | 36 | 1,5 g | 53 | 1,5 g | schwarz |
| 102 | 16 | 1,8 g | 52 | 1,2 g | schwarz |
| 103 | 17 | 2,4 g | 47 | 0,6 g | rotsrichiges schwarz |
| 104 | 18 | 1,5 g | 56 | 1,5 g | schwarz |

**Herstellung von wärmeempfindlichen Aufzeichnungsmaterialien**

**Beispiel 105**

In einer Kugelmühle werden 32 g 4,4'-Isopropyliden-diphenol (Bisphenol A), 3,8 g Distearylamid des Ethylendiamins, 89 g Kaolin, 20 g eines zu 88 % hydrolysierten Polyvinylalkohols und 55 ml Wasser gemahlen, bis die Teilchengröße ca. 5 µm beträgt. In einer zweiten Kugelmühle werden 6 g der 3,1-Benzoxazin-Verbindung des Beispiels 11, 3 g eines zu 88 % hydrolysierten Polyvinylalkohols und 60 ml Wasser zu einer Teilchengröße von ca. 3 µm gemahlen. Die beiden Dispersionen werden zusammengegeben und mit einem Trockenauftragsgewicht von 5,5 g/$m^2$ auf ein papier gestrichen. Durch Berühren des Papiers mit einem erhitzten Kugelschreiber wird eine intensive grüne Farbe erhalten, die eine gute Licht- und Sublimierechtheit hat.

**Beispiel 106**

In einer Kugelmühle werden 2,7 g der 3,1-Benzoxazin-Verbindung des Beispiels 44, 24 g N-Phenyl-N'-(1-hydroxy-2,2,2-trichlorethyl)-harnstoff, 16 g Stearinsäure, 59 g eines zu 88 % hydrolyiserten Polyvinylalkohols und 58 ml Wasser gemahlen, bis die Teilchengröße 2 bis 5 µm beträgt. Diese Suspension wird bei einem Trockenauftragsgewicht von 5,5 g/$m^2$ auf ein Blatt Papier gestrichen. Durch Berührung des Papiers mit einem erhitzten Kugelschreiber wird eine intensive und lichtechte Bordofarbe erhalten.

**Beispiel 107**

Nach DE-OS-3 337 296 werden in einer Kugelmühle 40 g eines feinpulvrigen Polyacrylnitrilpolymerisats, hergestellt aus 94 % Acrylnitril, 0,5 % Methallylsulfonsäure und 5,5 % Acrylsäuremethylester, mit 225 g einer 8 %-igen wäßrige Polyvinylalkohollösung und unter Zusatz von 1,3 g Distearylphosphorsäureester vermahlen. Eine zweite Dispersion wird aus 1 g der Benzoxazin-Verbindung des Beispiels 53 und 55 g einer 8 %-igen wäßrigen Polyvinylalkohollösung hergestellt. Man mischt die Dispersion des Farbbildners mit der des Akzeptors im Verhältnis 1/10 und trägt die Mischung auf Cellulosepapier mittels einer Rakel auf und trocknet sie, so daß man ein Auftragsgewicht von 6 bis 7 g/$m^2$ erhält. Das Papier ist mit Schreibmitteln, wie z. B. einem Kugelschreiber, beschriftbar. Es ist gegen starken Druck unempfindlich. Bei Berührung des Papiers mit einem beheizten Stift erhält man eine klare, scharfe, schattenfreie, schwarze Schrift. Die Lichtechtheit der Färbung ist ausgezeichnet.

**Patentansprüche**

1. Chromogene 3,1-Benzoxazine der Formel

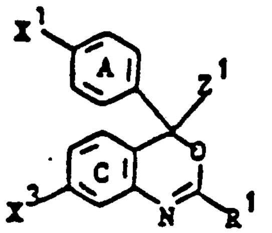

worin

$Z^1$ Wasserstoff, Alkyl, Cycloalkyl oder 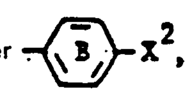

$R^1$ Wasserstoff, Alkyl, Cycloalkyl, Aralkyl, Aryl oder gegebenenfalls über Methylen oder Ethylen gebundenes Heteryl,

$X^1$, $X^2$ und $X^3$ unabhängig voneinander Wasserstoff, Halogen, Alkyl, Cycloalkyl, Aryl, Alkanoylamino, Aroylamino, Heteryl, $NY^1Y^2$, $OY^3$ oder $SY^3$, wobei wenigstens einer der Reste

$X^1$, $X^2$ oder $X^3$ für $NY^1Y^2$, $OY^3$ oder $SY^3$ steht,

$Y^1$, $Y^2$ und $Y^3$ unabhängig voneinander Wasserstoff, Alkyl, Cycloalkyl, Aralkyl, Aryl oder Heteryl oder die restlichen Glieder eines zu einem der o-ständigen Benzol-C-Atome reichenden, gegebenenfalls weitere Heteroatome enthaltenden 5- oder 6-gliedrigen Ringes oder

$Y^1 + Y^2$ die restlichen Glieder eines gegebenenfalls weitere Heteroatome enthaltenden 5- oder 6-gliedrigen Ringes bedeuten und

die Ringe A, B und C sowie die genannten Reste ihrerseits in der Farbstoffchemie übliche nichtionische Reste tragen oder die im Falle der Ringe A, B und C benzanelliert sein können,

und worin, wenn die Ringe A und B keine weiteren Substituenten und Anellierungen tragen und

- wenn $X^3$ für Wasserstoff, Dialkylamino oder Diaralkylamino steht -

$X^1$ und $X^2$ nicht gleichzeitig Dimethylamino sind, oder

- wenn $R^1$ für 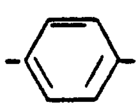 $OCH_3$ steht -

$X^1$ und $X^2$ nicht gleichzeitig Methoxy sind, oder

- wenn $Z^1$ für Wasserstoff steht -

$X^1$ nicht $C_1$- bis $C_3$-Alkoxy ist.

2. Chromogene 3,1-Benzoxazine der Formel

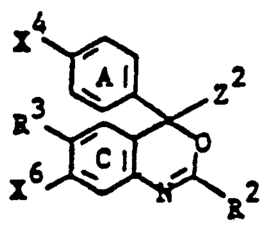

worin

einer der Reste

$X^6$ oder $R^3$ $NY^4Y^5$, $OY^6$ oder $SY^6$ und der andere Wasserstoff, $C_1$-$C_{18}$-Alkyl, das durch Fluor, Chlor, Brom, $C_1$-$C_8$-Alkoxy, Cyano und/oder $C_1$-$C_{18}$-Alkoxycarbonyl substituiert sein kann, Cyclohexyl, Benzyl-, Phenyl-, Biphenylyl- oder Naphthylreste, die durch Chlor, $C_1$-$C_8$-Alkyl, $C_1$-$C_8$-Alkoxy, Cyano, $C_1$-$C_4$-Alkoxycarbonyl und/oder $C_1$-$C_4$-Alkanoylamino substituiert sein können, oder $C_1$-$C_8$-Alkanoylamino oder Benzoylamino, das durch Chlor, $C_1$-$C_4$-Alkyl und/oder $C_1$-$C_4$-Alkoxy substituiert sein kann,

$Z^2$ Wasserstoff, $C_1$-$C_8$-Alkyl, Cyclohexyl oder 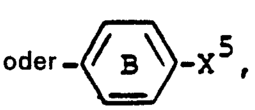

$R^2$ Wasserstoff, gegebenenfalls Fluor, Chlor, Brom, $C_1$-$C_8$-Alkoxy, Cyano oder $C_1$-$C_{18}$-Alkoxycarbonyl tragendes $C_1$-$C_{18}$-Alkyl, Cyclohexyl, gegebenenfalls Fluor, Chlor, Brom, Nitro, $C_1$-$C_{18}$-Alkyl, $C_1$-$C_{18}$-Alkoxy, $C_1$-$C_{18}$-Alkylthio, $C_1$-$C_{18}$-Mono- oder Dialkylamino, $C_1$-$C_{18}$-Alkylsulfonyl, Cyano, $C_1$-$C_{18}$-Alkoxycarbonyl und/oder $C_1$-$C_{18}$-Alkanoylamino tragende Benzyl-, Phenyl-, Biphenylyl-, Terphenylyl-, Naphthyl-, Picolyl-, Pyridyl-, Chinolyl-, Pyrimidyl-, Pyrazinyl-, Triazinyl-, Triazolyl-, Triazolylmethyl-, Thiadiazolyl-, Tetrazolyl-, Indolyl-,

gegebenenfalls benzanellierte Imidazol-, Oxazol- oder Thiazolreste,

$X^4$ und $X^5$ unabhängig voneinander Wasserstoff, Fluor, Chlor, Brom, gegebenenfalls durch Fluor, Chlor, Nitro, Hydroxy, $C_1$-$C_8$-Alkoxy, $C_1$-$C_8$-Alkylthio, Cyano, $C_1$-$C_8$-Alkoxycarbonyl, $C_1$-$C_8$-Alkanoyloxy, Amino und/oder Mono- oder Di-$C_1$-$C_4$-Alkylamino substituiertes $C_1$-$C_{18}$-Alkyl, gegebenenfalls durch Chlor und/oder $C_1$-$C_{12}$-Alkyl substituiertes Phenyl, $C_1$-$C_{12}$-Alkanoylamino, gegebenenfalls durch Chlor und/oder $C_1$-$C_{12}$-Alkyl substituiertes Benzoylamino, gegebenenfalls durch $C_1$-$C_4$-Alkyl, $C_1$-$C_4$-Alkoxy, Chlor und/oder Phenyl substituierte Indolyl- oder Piperidylreste, $NY^4Y^5$, $OY^6$ oder $SY^6$,

wobei aber höchstens zwei der Reste $X^4$, $X^5$ und $X^6$ $NY^4Y^5$ sind,

$Y^4$, $Y^5$ und $Y^6$ unabhängig voneinander Wasserstoff, gegebenenfalls durch Chlor, Hydroxy, Di-$C_1$-$C_4$-alkylamino, Cyano, $C_1$-$C_4$-Alkoxycarbonyl, $C_1$-$C_4$-Alkoxycarbonyloxy, $C_1$-$C_4$-Alkanoyloxy oder $C_1$-$C_4$-Alkoxy substituiertes $C_1$-$C_{18}$-Alkyl, Cyclohexyl oder Phenyl oder Benzyl, die durch Chlor, $C_1$-$C_{12}$-Alkyl oder $C_1$-$C_{12}$-Alkoxy substituiert sein können, gegebenenfalls durch $C_1$-$C_4$-Alkyl substituiertes Piperidyl oder Glieder die zusammen mit N oder O, an die sie gebunden sind und einem der Ringe A, B oder C zur Vervollständigung eines Ringsystems der folgenden Formeln

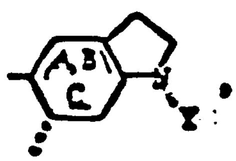,
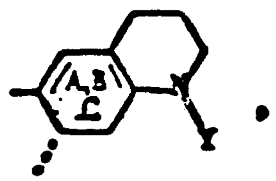,
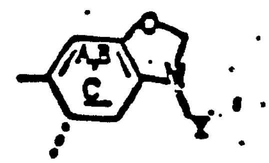,
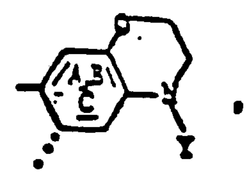,

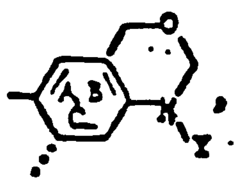,
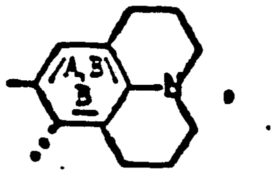,
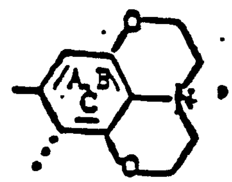,
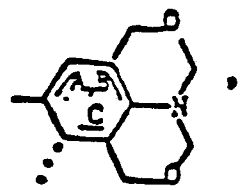,

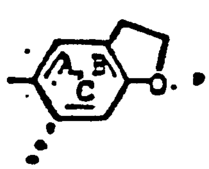,
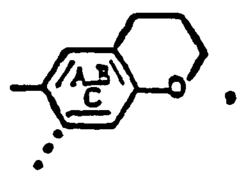,
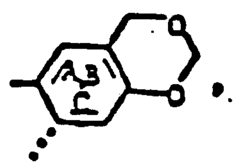,
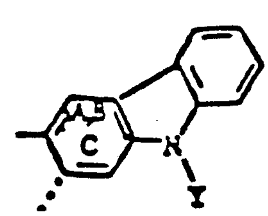

erforderlich sind, bedeuten, worin

die gestrichelte Linie die weitere Anellierung im Fall von Ring C bedeutet,

Y für Wasserstoff, $C_1$- bis $C_{18}$-Alkyl, das durch Chlor, Cyano, $C_1$- bis $C_4$-Alkoxycarbonyl oder $C_1$- bis $C_4$-Alkoxy substituiert sein kann, Cyclohexyl, Phenyl oder Benzyl, die durch Chlor, $C_1$- bis $C_{12}$-Alkyl oder $C_1$- bis $C_{12}$-Alkoxy substituiert sein können, steht,

der gesättigte Ringteil bis zu 4 Reste aus der Gruppe Chlor, $C_1$- bis $C_4$-Alkyl, $C_1$- bis $C_4$-Alkoxy oder Phenyl tragen kann,

die Ringe A, B und C durch Chlor, $C_1$-$C_4$-Alkyl, $C_1$-$C_4$-Alkoxy, gegebenenfalls durch Chlor, $C_1$-$C_4$-Alkyl oder $C_1$-$C_4$-Alkoxy substituiertes Phenoxy oder Phenylamino und/oder $C_1$-$C_4$-Alkanoylamino substituiert oder benzanelliert sein können, oder

$NY^4Y^5$ einen gegebenenfalls durch Chlor, $C_1$-$C_4$-Alkyl oder Aryl, insbesondere Phenyl substituierten Pyrrolo-, Pyrrolidino-, Piperidino-, Pipecolino-, Pyrazino-, Morpholino-, Pyrazolo- oder Pyrazolinorest bedeuten.

3. Chromogene 3,1-Benzoxazine der Formel

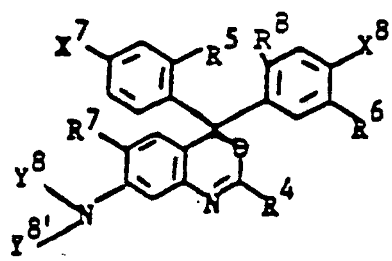

worin

$X^8$ Wasserstoff, Chlor, Brom, gegebenenfalls durch Chlor und/oder $C_1$-$C_4$-Alkoxy substituiertes $C_1$-$C_{18}$-Alkyl, gegebenenfalls durch Chlor und/oder $C_1$-$C_4$-Alkyl substituiertes Phenyl, $C_1$-$C_4$-Alkanoylamino, gegebenenfalls durch Chlor und/oder $C_1$-$C_4$-Alkyl substituiertes Benzoylamino, gegebenenfalls durch Methyl, Ethyl, Methoxy, Chlor und/oder Phenyl substituierte Indolyl- oder Piperidylreste, $OY^9$ oder $SY^9$,

$X^7$ $NY^7Y^{7'}$ oder unabhängig von $X^8$ die bei $X^8$ angegebenen Reste,

$R^4$ Wasserstoff, gegebenenfalls Fluor, Chlor oder $C_1$-$C_4$-Alkoxy tragendes $C_1$-$C_{18}$-Alkyl, Cyclohexyl, gegebenenfalls Chlor und/oder $C_1$-$C_8$-Alkyl tragendes Benzyl, gegebenenfalls Chlor, Brom, Nitro, $C_1$-$C_8$-Alkyl, $C_1$-$C_8$-Alkoxy, $C_1$-$C_8$-Alkylthio, $C_1$-$C_8$-Dialkylamino, $C_1$-$C_8$-Alkylsulfonyl, Cyano, $C_1$-$C_8$-Alkoxycarbonyl und/oder $C_1$-$C_8$-Alkanoylamino tragende Phenyl-, Biphenylyl-, Naphthyl-, Picolyl-, Pyridyl-, Chinolyl-, Pyrimidyl-, Pyrazinyl-, Triazolyl-, Thiadiazolyl-, Indolyl-, gegebenenfalls benzanellierte Imidazol-, Oxazol- oder Thiazolreste,

$R^5$, $R^6$, $R^7$ und $R^8$ unabhängig voneinander Wasserstoff, Chlor, Brom, $C_1$-$C_4$-Alkyl, $C_1$-$C_4$-Alkoxy, $C_1$-$C_4$-Alkylthio, Amino, $C_1$-$C_4$-Mono- oder Dialkylamino, $C_1$-$C_4$-Alkanoylamino, $C_1$-$C_4$-Alkylsulfonylamino, gegebenenfalls durch Methyl, Methoxy, Ethoxy oder Chlor substituierte Benzoylamino-, Anilino- oder N-$C_1$-$C_4$-Alkylanilinoreste,

$Y^7$ bis $Y^9$ unabhängig voneinander gegebenenfalls durch Chlor, Cyano, Methoxycarbonyl, Methoxycarbonyloxy, Acetyloxy, Hydroxy, Methoxy, Ethoxy oder Dimethylamino substituiertes $C_1$-$C_8$-Alkyl, Cyclohexyl, Benzyl, Phenyl, das durch Chlor, Cyano, Methyl, Ethyl, Methoxy oder Ethoxy substituiert sein kann, 2,2,6,6-Tetramethyl- oder 1,2,2,6,6-Pentamethylpiperidin-4-yl bedeuten,

$Y^{7'}$ und $Y^{8'}$ Wasserstoff bedeuten oder die Bedeutung von $Y^7$ bzw. $Y^8$ besitzen, oder

$NY^7Y^{7'}$ und $NY^8Y^{8'}$ unabhängig voneinander für einen durch $C_1$-$C_4$-Alkyl und/oder Phenyl, das noch Chlor, Methyl, Methoxy, Ethoxy oder Cyano tragen kann, substituierten Pyrrolidino-, Piperidino-, Piperazino-, Morpholino- oder Pyrazolinorest stehen.

4. Chromogene 3,1-Benzoxazine der Formel

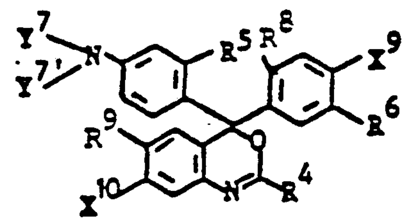

worin

$X^9$ Wasserstoff, Chlor, Brom, gegebenenfalls durch Chlor und/oder $C_1$-$C_4$-Alkoxy substituiertes $C_1$-$C_{18}$-Alkyl, gegebenenfalls durch Chlor und/oder $C_1$-$C_4$-Alkyl substituiertes Phenyl, $C_1$-$C_4$-Alkanoylamino, gegebenenfalls durch Chlor und/oder $C_1$-$C_4$-Alkyl substituiertes Benzoylamino, gegebenenfalls durch Methyl, Ethyl, Methoxy, Chlor und/oder Phenyl substituierte Indolyl- oder Piperidylreste, $NY^{10}Y^{10'}$, $OY^9$ oder $SY^9$,

$X^{10}$ $OY^{11}$ oder $SY^{11}$ und

$R^9$ Wasserstoff, Chlor, Brom, $C_1$-$C_4$-Alkyl, $C_1$-$C_4$-Alkoxy, $C_1$-$C_4$-Alkylthio, $C_1$-$C_4$-Alkanoylamino, Benzoylamino oder $C_1$-$C_4$-Alkylsulfonylamino oder

$X^{10}$ Wasserstoff, Chlor, Brom, gegebenenfalls durch Chlor und/oder $C_1$-$C_4$-Alkoxy substituiertes $C_1$-$C_{18}$-Alkyl, gegebenenfalls durch Chlor und/oder $C_1$-$C_4$-Alkyl substituiertes Phenyl, $C_1$-$C_4$-Alkanoylamino, gegebenenfalls durch Chlor und/oder $C_1$-$C_4$-Alkyl substituiertes Benzoylamino, gegebenenfalls durch Methyl, Ethyl, Methoxy, Chlor und/oder Phenyl substituierte Indolyl- oder Piperidylreste und

$R^9$ $C_1$-$C_4$-Alkoxy, $C_1$-$C_4$-Alkylthio, Amino, $C_1$-$C_4$-Mono- oder Dialkylamino oder gegebenenfalls durch Methyl, Methoxy, Ethoxy oder Chlor substituiertes Anilino oder N-$C_1$-$C_4$-Alkylanilino bedeuten,

$R^4$, $R^5$, $R^6$ und $R^8$ die in Anspruch 3 angegebene Bedeutung und

$Y^7$, $Y^{7'}$, $Y^9$ bis $Y^{11}$, $Y^{10'}$, $NY^7Y^{7'}$ und $NY^{10}Y^{10'}$ die in Anspruch 3 für $Y^7$, $Y^{7'}$ bzw. $NY^7Y^{7'}$ angegebene Bedeutung besitzen.

5. Chromogene 3,1-Benzoxazine der Formel

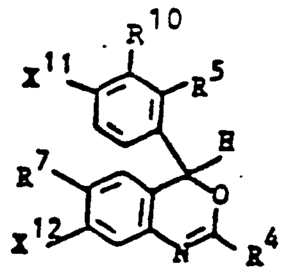

worin
$X^{11}$ und $X^{12}$ $NY^{12}Y^{12'}$, $OY^{13}$ oder $SY^{13}$ bedeuten und
$R^4$, $R^5$ und $R^7$ die in Anspruch 3 angegebene Bedeutung,
$R^{10}$ die in Anspruch 3 für $R^6$ angegebene Bedeutung und
$Y^{12}$, $Y^{12'}$ $Y^{13}$ und $NY^{12}Y^{12'}$ die Anspruch 3 für $Y^7$, $Y^{7'}$ bzw. $NY^7Y^{7'}$ angegebene Bedeutung besitzen.

6. Leukoverbindungen der Formel

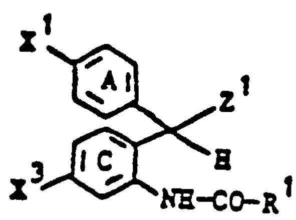

worin
$R^1$, $Z^1$, $X^1$, $X^2$, $X^3$, A, B und C die in Anspruch 1 angegebene Bedeutung besitzen und
wenn $Z^1$ für Wasserstoff steht und die Ringe A und C keine weiteren Substituenten oder anellierte Ringe tragen, $X^1$ und $X^3$ nicht gleichzeitig für 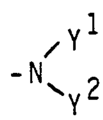 stehen.

7. Verfahren zur Herstellung eines 3,1-Benzoxazins des Anspruchs 1, dadurch gekennzeichnet daß man ein Amid der Formel

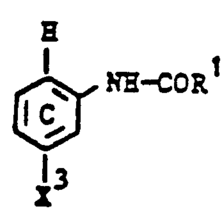

mit einem Keton oder Aldehyd der Formel

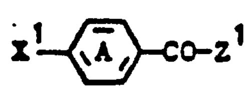

oder eine aromatische Verbindung der Formel

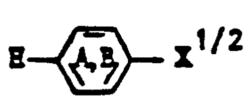

mit einem Keton der Formel

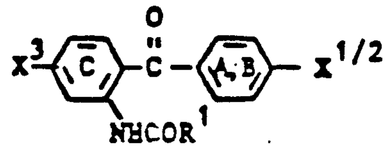

umsetzt, worin

$R^1$, $Z^1$, $X^1$, $X^2$, $X^3$, A, B und C die in Anspruch 1 angegebene Bedeutung besitzen.

8. Verfahren zur Herstellung eines 3,1-Benzoxazins des Anspruchs 1, dadurch gekennzeichnet, daß man eine Leukoverbindung der Formel

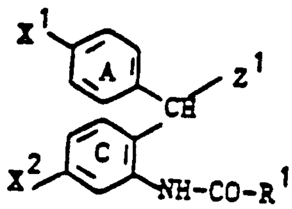

oxidiert, worin

$Z^1$, $X^1$, $X^2$, $X^3$, $R^1$, A, B und C die in Anspruch 1 angegebene Bedeutung besitzen.

9. Verwendung chromogener 3,1-Benzoxazine des Anspruchs 1 für druckkopierfähige, thermoreaktive oder elektrochrome Aufzeichnungsmaterialien, die einen sauren Farbentwickler enthalten.

10. Druck-, wärme- und elektrosensitives Aufzeichnungsmaterial, dadurch gekennzeichnet, daß es in seinem Reaktantensystem ein 3,1-Benzoxazin der in Anspruch 1 angegebenen Formel als Farbbildner und einen sauren Farbentwickler enthält.

## Claims

1. Chromogenic 3,1-benzoxazines of the formula

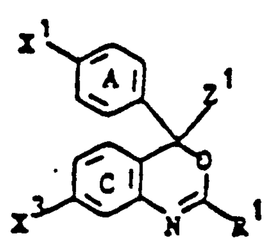

wherein

$Z^1$ denotes hydrogen, alkyl, cycloalkyl or 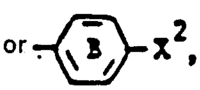

$R^1$ denotes hydrogen, alkyl, cycloalkyl, aralkyl, aryl, or heteryl, which is optionally bonded via methylene or ethylene,

$X^1$, $X^2$ and $X^3$ independently of one another denote hydrogen, halogen, alkyl, cycloalkyl, aryl, alkanoylamino, aroylamino, heteryl, $NY^1Y^2$, $OY^3$ or $SY^3$, at least one of the radicals $X^1$, $X^2$ or $X^3$ representing $NY^1Y^2$, $OY^3$ or $SY^3$,

$Y^1$, $Y^2$ and $Y^3$ independently of one another denote hydrogen, alkyl, cycloalkyl, aralkyl, aryl or heteryl or the remaining members of a 5-membered or 6-membered ring which extends to one of the benzene-C atoms in the o-position and optionally contains further heteroatoms, or

$Y^1 + Y^2$ denote the remaining members of a 5-membered or 6-membered ring which optionally contains further heteroatoms and

the rings A, B and C and the radicals mentioned can in turn carry nonionic radicals which are customary in dyestuff chemistry, or the rings A, B and C can be benzo-fused, and wherein, if the rings A and B do not carry further substituents or fused-on systems and

- if $X^3$ represents hydrogen, dialkylamino or diaralkylamino -

$X^1$ and $X^2$ are not simultaneously dimethylamino, or

- if $R^1$ represents 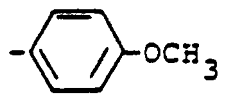 -

$X^1$ and $X^2$ are not simultaneously methoxy, or
- if $Z^1$ represents hydrogen -
$X^1$ is not $C_1$- to $C_3$-alkoxy.

2. Chromogenic 3,1-benzoxazines of the formula

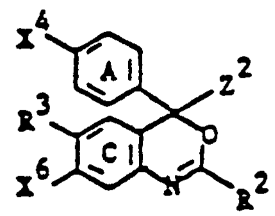

wherein

one of the radicals $X^6$ or $R^3$ denotes $NY^4Y^5$, $OY^6$ or $SY^6$ and the other denotes hydrogen, $C_1$-$C_{18}$-alkyl, which can be substituted by fluorine, chlorine, bromine, $C_1$-$C_8$-alkoxy, cyano and/or $C_1$-$C_{18}$-alkoxycarbonyl, cyclohexyl, or benzyl, phenyl, biphenylyl or naphthyl radicals which can be substituted by chlorine, $C_1$-$C_8$-alkyl, $C_1$-$C_8$-alkoxy, cyano, $C_1$-$C_4$-alkoxycarbonyl and/or $C_1$-$C_4$-alkanoylamino, or $C_1$-$C_8$-alkanoylamino or benzoylamino, which can be substituted by chlorine, $C_1$-$C_4$-alkyl and/or $C_1$-$C_4$-alkoxy,

$Z^2$ denotes hydrogen, $C_1$-$C_8$-alkyl, cyclohexyl or

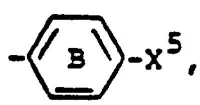,

$R^2$ denotes hydrogen, $C_1$-$C_{18}$-alkyl which optionally carries fluorine, chlorine, bromine, $C_1$-$C_8$-alkoxy, cyano or $C_1$-$C_{18}$-alkoxycarbonyl, cyclohexyl, or benzyl, phenyl, biphenylyl, terphenylyl, naphthyl, picolyl, pyridyl, quinolyl, pyrimidyl, pyrazinyl, triazinyl, triazolyl, triazolylmethyl, thiadiazolyl, tetrazolyl, indolyl or optionally benzofused imidazole, oxazole or thiazole radicals which optionally carry fluorine, chlorine, bromine, nitro, $C_1$-$C_{18}$-alkyl, $C_1$-$C_{18}$-alkoxy, $C_1$-$C_{18}$-alkylthio, $C_1$-$C_{18}$-mono- or dialkylamino, $C_1$-$C_{18}$-alkylsulphonyl, cyano, $C_1$-$C_{18}$-alkoxycarbonyl and/or $C_1$-$C_{18}$-alkanoylamino,

$X^4$ and $X^5$ independently of one another denote hydrogen, fluorine, chlorine, bromine or $C_1$-$C_{18}$-alkyl which is optionally substituted by fluorine, chlorine, nitro, hydroxyl, $C_1$-$C_8$-alkoxy, $C_1$-$C_8$-alkylthio, cyano, $C_1$-$C_8$-alkoxycarbonyl, $C_1$-$C_8$-alkanoyloxy, amino and/or mono- or di-$C_1$-$C_4$-alkylamino, phenyl which is optionally substituted by chlorine and/or $C_1$-$C_{12}$-alkyl, $C_1$-$C_{12}$-alkanoylamino, benzoylamino which is optionally substituted by chlorine and/or $C_1$-$C_{12}$-alkyl, indolyl or piperidyl radicals which are optionally substituted by $C_1$-$C_4$-alkyl, $C_1$-$C_4$-alkoxy, chlorine and/or phenyl, $NY^4Y^5$ $OY^6$ or $SY^6$,

but wherein at most two of the radicals $X^4$, $X^5$ and $X^6$ are $NY^4Y^5$,

$Y^4$, $Y^5$ and $Y^6$ independently of one another denote hydrogen, $C_1$-$C_{18}$-alkyl which is optionally substituted by chlorine, hydroxyl, di-$C_1$-$C_4$-alkylamino, cyano, $C_1$-$C_4$-alkoxycarbonyl, $C_1$-$C_4$-alkoxycarbonyloxy, $C_1$-$C_4$-alkanoyloxy or, $C_1$-$C_4$-alkoxy, cyclohexyl or phenyl or benzyl, which can be substituted by chlorine, $C_1$-$C_{12}$-alkyl or $C_1$-$C_{12}$-alkoxy, piperidyl which is optionally substituted by $C_1$-$C_4$-alkyl, or members which, together with the N or O to which they are bonded and one of the rings A, B or C, are necessary to complete a ring system of the following formulae

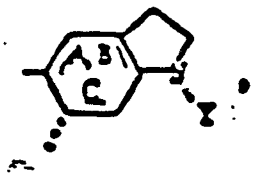 , 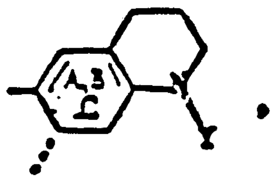 , 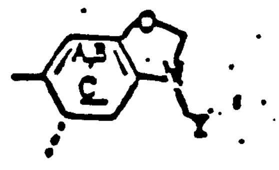 , 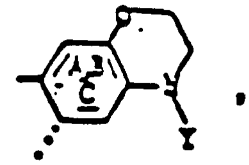 ,

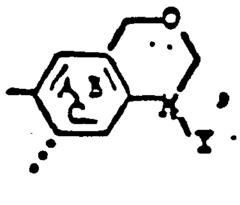 , 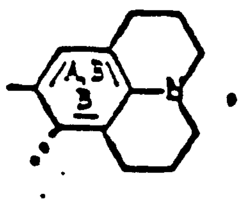 , 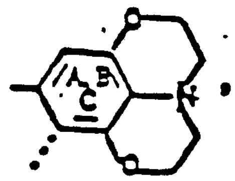 , 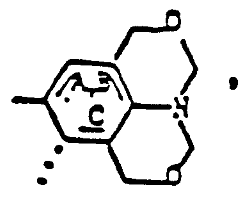 ,

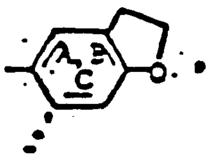 , 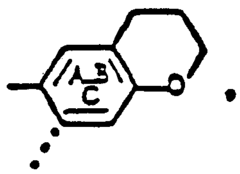 , 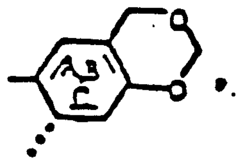 , 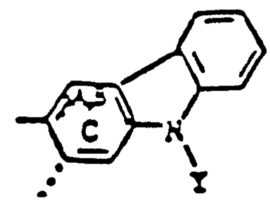

wherein

the broken line denotes further fusing-on in the case of ring C,

Y represents hydrogen, $C_1$- to $C_{18}$-alkyl, which can be substituted by chlorine, cyano, $C_1$- to $C_4$-alkoxycarbonyl or $C_1$- to $C_4$-alkoxy, cyclohexyl, phenyl or benzyl, which can be substituted by chlorine, $C_1$- to $C_{12}$-alkyl or $C_1$- to $C_{12}$-alkoxy,

the saturated ring part can carry up to 4 radicals from the group comprising chlorine, $C_1$- to $C_4$-alkyl, $C_1$- to $C_4$-alkoxy and phenyl,

the rings A, B and C can be substituted by chlorine, $C_1$-$C_4$-alkyl, $C_1$-$C_4$-alkoxy, phenoxy or phenylamino which is optionally substitutted by chlorine, $C_1$-$C_4$-alkyl or $C_1$-$C_4$-alkoxy and/or $C_1$-$C_4$-alkanoylamino or benzo-fused, or

$NY^4Y^5$ denotes a pyrrolo, pyrrolidino, piperidino, pipecolino, pyrazino, morpholino, pyrazolo or pyrazolino radical which is optionally substituted by chlorine, $C_1$-$C_4$-alkyl or aryl, in particular phenyl.

3. Chromogenic 3,1-benzoxazines of the formula

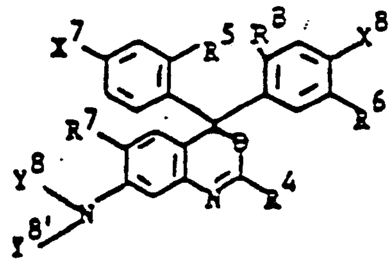

wherein

$X^8$ denotes hydrogen, chlorine, bromine, $C_1$-$C_{18}$-alkyl which is optionally substituted by chlorine and/or $C_1$-$C_4$-alkoxy, phenyl which is optionally substituted by chlorine and/or $C_1$-$C_4$-alkyl, $C_1$-$C_4$-alkanoylamino, benzoylamino which is optionally substituted by chlorine and/or $C_1$-$C_4$-alkyl, indolyl or piperidyl radicals which are optionally substituted by methyl, ethyl, methoxy, chlorine and/or phenyl, $OY^9$ or $SY^9$,

$X^7$ denotes $NY^7Y^{7'}$ or independently of $X^8$ denotes the radicals given in the case of $X^8$,

$R^4$ denotes hydrogen, $C_1$-$C_{18}$-alkyl which optionally carries fluorine, chlorine or $C_1$-$C_4$-alkoxy, cyclohexyl, benzyl which optionally carries chlorine and/or $C_1$-$C_8$-alkyl, or phenyl, biphenylyl, naphthyl, picolyl, pyridyl, quinolyl, pyrimidyl, pyrazinyl, triazolyl, thiadiazolyl, indolyl or optionally benzo-fused imidazole, oxazole or thiazole radicals which optionally carry chlorine, bromine, nitro, $C_1$-$C_8$-alkyl, $C_1$-$C_8$-alkoxy, $C_1$-$C_8$-alkylthio, $C_1$-$C_8$-dialkylamino, $C_1$-$C_8$-alkylsulphonyl, cyano, $C_1$-$C_8$-alkoxycarbonyl and/or or $C_1$-$C_8$-alkanoylamino,

$R^5$, $R^6$, $R^7$ and $R^8$ independently of one another denote hydrogen, chlorine, bromine, $C_1$-$C_4$-alkyl, $C_1$-$C_4$-alkoxy, $C_1$-$C_4$-alkylthio, amino, $C_1$-$C_4$-mono- or dialkylamino, $C_1$-$C_4$-alkanoylamino, or $C_1$-$C_4$-alkylsulphonylamino, or benzoylamino, anilino or N-$C_1$-$C_4$-alkylanilino radicals which are optionally substituted by methyl, methoxy, ethoxy or chlorine,

$Y^7$ to $Y^9$ independently of one another denote $C_1$-$C_8$-alkyl which is optionally substituted by chlorine, cyano, methoxycarbonyl, methoxycarbonyloxy, acetyloxy, hydroxyl, methoxy, ethoxy or dimethylamino, cyclohexyl, benzyl, phenyl, which can be substituted by chlorine, cyano, methyl, ethyl, methoxy or ethoxy, or 2,2,6,6-tetramethyl- or 1,2,2,6,6-pentamethylpiperidin-4-yl,

$Y^{7'}$ and $Y^{8'}$ denote hydrogen or have the meaning of $Y^7$ or $Y^8$ respectively, or

$NY^7Y^{7'}$ and $NY^8Y^{8'}$ independently of one another represent a pyrrolidino, piperidino, piperazino, morpholino or pyrazolino radical which is substituted by $C_1$-$C_4$-alkyl and/or phenyl, which can also carry chlorine, methyl, methoxy, ethoxy or cyano.

4. Chromogenic 3,1-benzoxazines of the formula

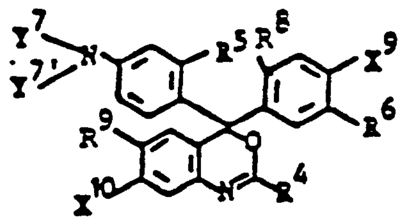

wherein

$X^9$ denotes hydrogen, chlorine, bromine, $C_1$-$C_{18}$-alkyl which is optionally substituted by chlorine and/or $C_1$-$C_4$-alkoxy, phenyl which is optionally substituted by chlorine and/or $C_1$-$C_4$-alkyl, $C_1$-$C_4$-alkanoylamino, benzoylamino which is optionally substituted by chlorine and/or $C_1$-$C_4$-alkyl, indolyl or piperidyl radicals which are optionally substituted by methyl, ethyl, methoxy, chlorine and/or phenyl, $NY^{10}Y^{10'}$, $OY^9$ or $SY^9$, $X^{10}$ denotes $OY^{11}$ or $SY^{11}$ and

$R^9$ denotes hydrogen, chlorine, bromine, $C_1$-$C_4$-alkyl, $C_1$-$C_4$-alkoxy, $C_1$-$C_4$-alkylthio, $C_1$-$C_4$-alkanoylamino, benzoylamino or $C_1$-$C_4$-alkylsulphonylamino, or

$X^{10}$ denotes hydrogen, chlorine, bromine, $C_1$-$C_{18}$-alkyl which is optionally substituted by chlorine and/or $C_1$-$C_4$-alkoxy, phenyl which is optionally substituted by chlorine and/or $C_1$-$C_4$-alkyl, $C_1$-$C_4$-alkanoylamino, benzoylamino which is optionally substituted by chlorine and/or $C_1$-$C_4$-alkyl, or indolyl or piperidyl radicals which are optionally substituted by methyl, ethyl, methoxy, chlorine and/or phenyl and

$R^9$ denotes $C_1$-$C_4$-alkoxy, $C_1$-$C_4$-alkylthio, amino or $C_1$-$C_4$-mono- or dialkylamino, or anilino or N-$C_1$-$C_4$-alkylanilino which is optionally substituted by methyl, methoxy, ethoxy or chlorine, $R^4$, $R^5$, $R^6$ and $R^8$ have the meaning given in Claim 3 and

$Y^7$, $Y^{7'}$, $Y^9$ to $Y^{11}$, $Y^{10'}$, $NY^7Y^{7'}$ and $NY^{10}Y^{10'}$ have the meaning given for $Y^7$, $Y^{7'}$ and $NY^7Y^{7'}$ respectively in Claim 3.

5. Chromogenic 3,1-benzoxazines of the formula

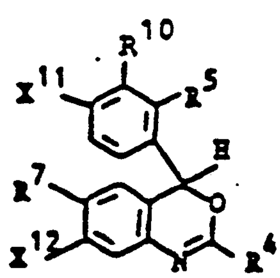

wherein

$X^{11}$ and $X^{12}$ denote $NY^{12}Y^{12'}$, $OY^{13}$ or $SY^{13}$ and $R^4$, $R^5$ and $R^7$ have the meaning given in Claim 3,

$R^{10}$ has the meaning given for $R^6$ in Claim 3 and $Y^{12}$, $Y^{12'}$, $Y^{13}$ and $NY^{12}Y^{12'}$ have the meaning given for $Y^7$, $Y^{7'}$ and $NY^7Y^{7'}$ respectively in Claim 3.

6. Leuco compounds of the formula

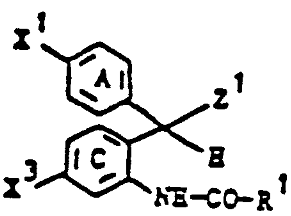

wherein

$R^1$, $Z^1$, $X^1$, $X^2$, $X^3$, A, B and C have the meaning given in Claim 1 and if $Z^1$ represents hydrogen and the rings A and C do not carry further substituents or fused-on rings,

$X^1$ and $X^3$ do not simultaneously represent 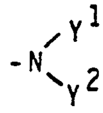 .

7. Process for the preparation of a 3,1-benzoxazine of Claim 1, characterized in that an amide of the formula

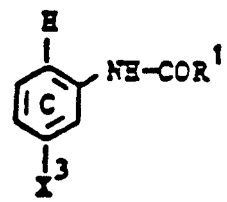

is reacted with a ketone or aldehyde of the formula

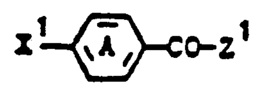

or an aromatic compound of the formula

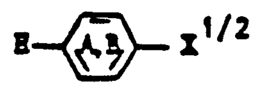 .

is reacted with a ketone of the formula

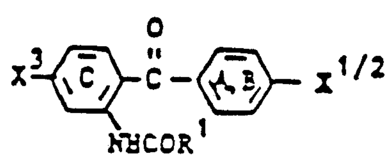

wherein

$R^1$, $Z^1$, $X^1$, $X^2$, $X^3$, A, B and C have the meaning given in Claim 1.

8. Process for the preparation of a 3,1-benzoxazine of Claim 1, characterized in that a leuco compound of the formula

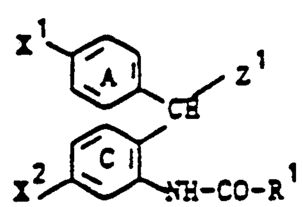

wherein

$Z^1$, $X^1$, $X^2$, $X^3$, $R^1$, A, B and C have the meaning given in Claim 1,

is oxidised.

9. Use of chromogenic 3,1-benzoxazines of Claim 1 for recording materials which are capable of pressure-copying or are thermoreactive or electrochromic, and contain an acid colour developer.

10. Pressure-sensitive, heat-sensitive and electro-sensitive recording material, characterized in that it contains, in its reactant system, a 3,1-benzoxazine of the formula given in Claim 1, as the colour-forming agent, and an acid colour developer.

**Revendications**

1. 3,1-benzoxazines chromogènes de formule

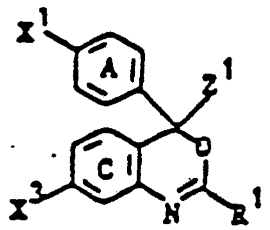

dans laquelle

$Z^1$ représente l'hydrogène, un groupe alkyle, cycloalkyle ou

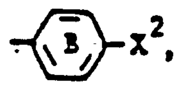

$R^1$ représente l'hydrogène, un groupe alkyle, cycloalkyle, aralkyle, aryle ou un groupe hétéryle éventuellement lié par un groupe méthylène ou éthylène,

$X^1$, $X^2$ et $X^3$ représentent, indépendamment l'un de l'autre, l'hydrogène, un halogène, un groupe alkyle, cycloalkyle, aryle, alcanoylamino, aroylamino, hétéryle, $NY^1Y^2$, $OY^3$ ou $SY^3$, au moins l'un des restes $X^1$, $X^2$ ou $X^3$ représentant un groupe $NY^1Y^2$, $OY^3$ ou $SY^3$,

$Y^1$, $Y^2$ et $Y^3$ représentent, indépendamment l'un de l'autre, l'hydrogène, un groupe alkyle, cycloalkyle, aralkyle, aryle ou hétéryle ou bien les groupes restants d'un noyau pentagonal ou hexagonal atteignant l'un des atomes de carbone en position ortho du benzène, contenant éventuellement d'autres hétéroatomes, ou bien

$Y^1 + Y^2$ représentent les membres restants d'un noyau pentagonal ou hexagonal contenant éventuellement d'autres hétéroatomes et

les noyaux A, B et C ainsi que les restes mentionnés pouvant porter quant à eux des restes non ioniques classiques dans la chimie des colorants ou pouvant être condensés au benzène dans le cas des noyaux A, B et C, et dans laquelle, lorsque les noyaux A et B ne portent pas d'autres substituants et d'autres condensations et

- lorsque $X^3$ représente l'hydrogène, un groupe dialkylamino ou diaralkylamino -

$X^1$ et $X^2$ ne sont pas simultanément des groupes diméthylamino, ou bien

- lorsque $R^1$ est un groupe 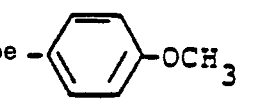 -

$X^1$ et $X^2$ ne sont pas simultanément des groupes méthoxy, ou bien

- lorsque $Z^1$ représente l'hydrogène -

$X^1$ n'est pas un groupe alkoxy en $C_1$ à $C_3$.

2. 3,1-benzoxazines chromogènes de formule

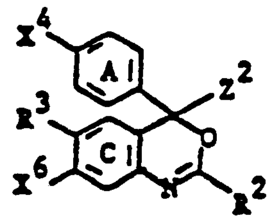

dans laquelle

l'un des restes

$X^6$ ou $R^3$ représente un groupe $NY^4Y^5$, $OY^6$ ou $SY^6$ et l'autre représente l'hydrogène, un groupe alkyle en $C_1$ à $C_{18}$ qui peut être substitué par du fluor, du chlore, du brome, un substituant alkoxy en $C_1$ à $C_8$, cyano et/ou (alkoxy en $C_1$ à $C_{18}$)-carbonyle, des restes cyclohexyle, benzyle, phényle, biphénylyle ou naphtyle qui peuvent être substitués par du chlore, un groupe alkyle en $C_1$ à $C_8$, alkoxy en $C_1$ à $C_8$, cyano, (alkoxy en $C_1$ à $C_4$) carbonyle et/ou alcanoylamino en $C_1$ à $C_4$, ou un groupe alcanoylamino en $C_1$ à $C_8$ ou benzoylamino qui peut être substitué par du chlore, un substituant alkyle en $C_1$ à $C_4$ et/ou alkoxy en $C_1$ à $C_4$,

$Z^2$ représente l'hydrogène, un groupe alkyle en $C_1$ à $C_8$, cyclohexyle ou

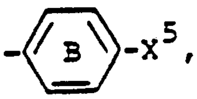

$R^2$ représente l'hydrogène, un groupe alkyle en $C_1$ à $C_{18}$ portant eventuellement du fluor, du chlore, du brome, un radical alkoxy en $C_1$ à $C_8$, cyano ou (alkoxy en $C_1$ à $C_{18}$)-carbonyle, un groupe cyclohexyle, des restes benzyle, phényle, biphénylyle, terphénylyle, naphtyle, picolyle, pyridyle, quinolyle, pyrimidyle, pyrazinyle, triazinyle, triazolyle, triazolylméthyle, thiadiazolyle, tétrazolyle, indolyle, imidazole, oxazole ou thiazole éventuellement condensés au benzène, portant le cas échéant du fluor, du chlore, du brome, des radicaux nitro, alkyle en $C_1$ à $C_{18}$, alkoxy en $C_1$ à $C_{18}$, alkylthio en $C_1$ à $C_{18}$, mono- ou di-(alkyle en $C_1$ à $C_{18}$)-amino, alkylsulfonyle en $C_1$ à $C_{18}$, cyano, (alkoxy en $C_1$ à $C_{18}$)-carbonyle et/ou alcanoylamino en $C_1$ à $C_{18}$,

$X^4$ et $X^5$ représentent, indépendamment l'un de l'autre, l'hydrogène, le fluor, le chlore, le brome, un groupe alkyle en $C_1$ à $C_{18}$ éventuellement substitué par du fluor, du chlore, un radical nitro, hydroxy, alkoxy en $C_1$ à $C_8$, alkylthio en $C_1$ à $C_8$, cyano, (alkoxy en $C_1$ à $C_8$)-carbonyle, alcanoyloxy en $C_1$ à $C_8$, amino et/ou mono- ou di-(alkyle en $C_1$ à $C_4$)-amino, un groupe phényle eventuellement substitué par du chlore et/ou un radical alkyle en $C_1$ à $C_{12}$, un groupe alcanoylamino en $C_1$ à $C_{12}$, un groupe benzoylamino éventuellement substitué par du chlore et/ou un radical alkyle en $C_1$ à $C_{12}$, des restes indolyle ou pipéridyle éventuellement substitués par des radicaux alkyle en $C_1$ à $C_4$, alkoxy en $C_1$ à $C_4$, du chlore et/ou le radical phényle, un groupe $NY^4Y^5$, $OY^6$ ou $SY^6$,

mais avec un maximum de deux des restes $X^4$, $X^5$ et $X^6$ représentant des groupes $NY^4Y^5$,

$Y^4$, $Y^5$ et $Y^6$ représentent, indépendamment l'un de l'autre, l'hydrogène, un groupe alkyle en $C_1$ à $C_{18}$ éventuellement substitué par du chlore, un radical hydroxy, di-(alkyle en $C_1$ à $C_4$)-amino, cyano, (alkoxy en $C_1$ à $C_4$)-carbonyle, (alkoxy en $C_1$ à $C_4$)-carbonyloxy, alcanoyloxy en $C_1$ à $C_4$ ou alkoxy en $C_1$ à $C_4$, un groupe cyclohexyle ou des groupes phényle ou benzyle qui peuvent être substitués par du chlore, des radicaux alkyle en $C_1$ à $C_{12}$ ou alkoxy en $C_1$ à $C_{12}$, un groupe pipéridyle éventuellement substitué par un radical alkyle en $C_1$ à $C_4$, ou des membres qui sont necessaires, conjointement avec l'azote ou l'oxygène auquel ils sont liés et l'un des noyaux A, B ou C, pour compléter un système de noyaux de formules suivantes:

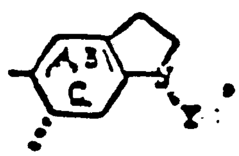, 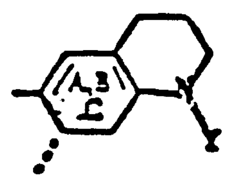, 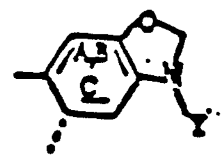, 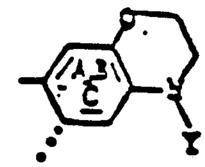,

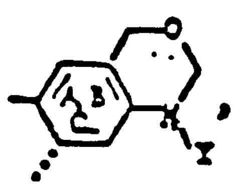, 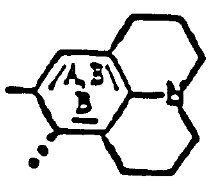, 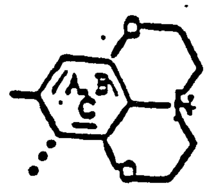, 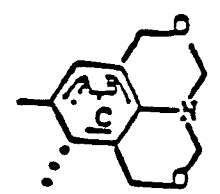,

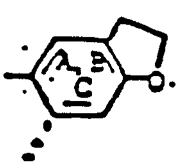, 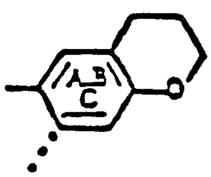, 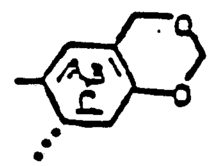, 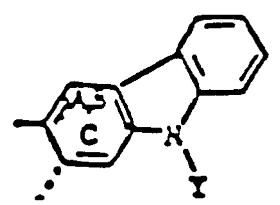

dans lesquelles

le segment en traits interrompus représente l'autre condensation dans le cas du noyau C,

Y est l'hydrogène, un groupe alkyle en $C_1$ à $C_{18}$ qui peut être substitué par du chlore, un radical cyano, (alkoxy en $C_1$ à $C_4$)-carbonyle ou alkoxy en $C_1$ à $C_4$, des groupes cyclohexyle, phényle ou benzyle qui peuvent être substitués par du chlore, des radicaux alkyle en $C_1$ à $C_{12}$ ou des radicaux alkoxy en $C_1$ à $C_{12}$,

la partie saturée du noyau pouvant porter jusqu'à 4 restes du groupe des restes chloro, alkyle en $C_1$ à $C_4$, alkoxy en $C_1$ à $C_4$ ou phényle,

les noyaux A, B et C pouvant être substitués par du chlore, un groupe alkyle en $C_1$ à $C_4$, alkoxy en $C_1$ à $C_4$, un groupe phénoxy ou phénylamino substitué le cas échéant par du chlore, un radical alkyle en $C_1$ à $C_4$ ou alkoxy en $C_1$ à $C_4$ et/ou un groupe alcanoylamino en $C_1$ à $C_4$ ou pouvant être condensés au benzène, ou bien

$NY^4Y^5$ représente un reste pyrrolo, pyrrolidino, pipéridino, pipécolino, pyrazino, morpholino, pyrazolo ou pyrazolino substitués le cas échéant par du chlore, un groupe alkyle en $C_1$ à $C_4$ ou aryle, notamment phényle.

3. 3,1-benzoxazines chromogènes de formule

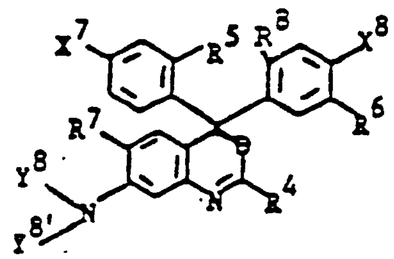

dans laquelle

$X^8$ représente de l'hydrogène, du chlore, du brome, un groupe alkyle en $C_1$ à $C_{18}$ éventuellement substitué par du chlore et/ou un radical alkoxy en $C_1$ à $C_4$, un groupe phényle éventuellement substitué par du chlore et/ou un radical alkyle en $C_1$ à $C_4$, un groupe alcanoylamino en $C_1$ à $C_4$, un groupe benzoylamino éventuellement substitué par du chlore et/ou par un radical alkyle en $C_1$ à $C_4$, des restes indolyle ou pipéridyle éventuellement substitués par des radicaux méthyle, éthyle, méthoxy, chloro et/ou phényle, un groupe $OY^9$ ou $SY^9$,

$X^7$ est un groupe $NY^7Y^{7'}$ ou, indépendamment de $X^8$, les restes indiqués dans le cas de $X^8$,

$R^4$ représente l'hydrogène, un groupe alkyle en $C_1$ à $C_{18}$ portant éventuellement du fluor, du chlore ou un radical alkoxy en $C_1$ à $C_4$, un groupe cyclohexyle, un groupe benzyle portant éventuellement du chlore et/ou un radical alkyle en $C_1$ à $C_8$, les restes phényle, biphénylyle, naphtyle, picolyle, pyridyle, quinolyle, pyrimidyle, pyrazinyle, triazolyle, thiadiazolyle, indolyle, des restes imidazole oxazole ou thiazole éventuellement condenses au benzène, portant le cas échéant du chlore, du brome, des radicaux nitro, alkyle en $C_1$ à $C_8$, alkoxy en $C_1$ à $C_8$, alkylthio en $C_1$ à $C_8$, dialkylamino en $C_1$ à $C_8$, alkylsulfonyle en $C_1$ à $C_8$, cyano, (alkoxy en $C_1$ à $C_8$)-carbonyle et/ou

$R^5$, $R^6$, $R^7$ et $R^8$ représentent, indépendamment les une des autres, l'hydrogène, le chlore, le brome, les groupes alkyle en $C_1$ à $C_4$, alkoxy en $C_1$ à $C_4$, alkylthio en $C_1$ à $C_4$, amino, mono- ou di-(alkyle en $C_1$ à $C_4$)-amino, alcanoylamino en $C_1$ à $C_4$, alkylsulfonylamino en $C_1$ à $C_4$, des restes benzoylamino, anilino ou N-(alkyle en $C_1$ à $C_4$)-anilino éventuellement substitués par les radicaux méthyle, méthoxy, éthoxy ou du chore,

$Y^7$ à $Y^9$ représentent, indépendamment l'un de l'autre, un groupe alkyle en $C_1$ à $C_8$ éventuellement substitué par un radical chloro, cyano, méthoxycarbonyle, méthoxycarbonyloxy, acétyloxy, hydroxy, méthoxy, éthoxy ou diméthylamino, un groupe cyclohexyle, un groupe benzyle, un groupe phényle qui peut être substitué par un radical chloro, cyano, méthyle, éthyle, méthoxy ou éthoxy, un reste 2,2,6,6-tétraméthyl- ou 1,2,2,6,6-pentaméthylpipéridine-4-yle,

$Y^{7'}$ et $Y^{8'}$ représentent l'hydrogène ou ont la définition de $Y^7$ et respectivement de $Y^8$, ou bien

$NY^7Y^{7'}$ et $NY^8Y^{8'}$ représentent, independamment l'un de l'autre, un reste pyrrolidino, pipéridino, pipérazino, morpholino ou pyrazolino éventuellement substitué par un radical alkyle en $C_1$ à $C_4$ et/ou phényle, qui peut porter en outre du chlore, un radical méthyle, méthoxy, éthoxy ou cyano.

4. 3,1-benzoxazir

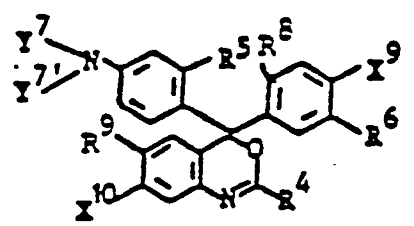

dans laquelle

$X^9$ représente l'hydrogène, le chlore, le brome, un groupe alkyle en $C_1$ à $C_{18}$ éventuellement substitué par du chlore et/ou un radical alkoxy en $C_1$ à $C_4$, un groupe phényle éventuellement substitué par du chlore et/ou un radical alkyle en $C_1$ à $C_4$, un groupe alcanoylamino en $C_1$ à $C_4$, un groupe benzoylamino éventuellement substitué par du chlore et/ou un radical alkyle en $C_1$ à $C_4$, des restes indolyle ou pipéridyle éventuellement substitués par des radicaux méthyle, éthyle, méthoxy, chloro et/ou phényle, un groupe $NY^{10}Y^{10'}$, $OY^9$ ou $SY^9$,

$X^{10}$ représente $OY^{11}$ ou $SY^{11}$ et

$R^9$ représente l'hydrogène, le chlore, le brome, un groupe alkyle en $C_1$ à $C_4$, alkoxy en $C_1$ à $C_4$, benzoylamino ou alkylsulfonylamino en $C_1$ à $C_4$, ou bien

$X^{10}$ représente l'hydrogène, le chlore, le brome, un groupe alkyle en $C_1$ à $C_{18}$ éventuellement substitué par du chlore et/ou par un radical alkoxy en $C_1$ à $C_4$, un groupe phényle éventuellement substitué par du chlore et/ou un radical alkyle en $C_1$ à $C_4$, un groupe alcanoylamino en $C_1$ à $C_4$, un groupe benzoylamino éventuellement substitué par du chlore et/ou un radical alkyle en $C_1$ à $C_4$, des restes indolyle ou pipéridyle éventuellement substitués par des radicaux méthyle, éthyle, méthoxy, chloro et/ou phényle et

$R^9$ est un groupe alkoxy en $C_1$ à $C_4$, alkylthio en $C_1$ à $C_4$, amino, mono- ou di-(alkyle en $C_1$ à $C_4$)-amino ou un groupe anilino ou N-(alkyle en $C_1$ à $C_4$)-anilino éventuellement substitué par un radical méthyle, méthoxy, éthoxy ou chloro,

$R^4$, $R^5$, $R^6$ et $R^8$ ont la définition indiquée dans la revendication 3 et

$Y^7$, $Y^{7'}$, $Y^9$ à $Y^{11}$, $Y^{10'}$, $NY^7Y^{7'}$ et $NY^{10}Y^{10'}$ ont la définition indiquée dans la revendication 3 pour $Y^7$, $Y^{7'}$ et respectivement $NY^7Y^{7'}$.

5. 3,1-benzoxazines chromogènes de formule

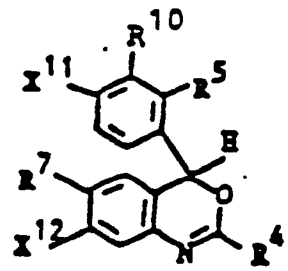

dans laquelle

$X^{11}$ et $X^{12}$ représentent $NY^{12}Y^{12'}$, $OY^{13}$ ou $SY^{13}$ et

$R^4$, $R^5$ et $R^7$ ont la définition indiquée dans la revendication 3,

$R^{10}$ a la definition indiquée pour $R^6$ dans la revendication 3 et

$Y^{12}$, $Y^{12'}$, $Y^{13}$ et $NY^{12}Y^{12'}$ ont la définition indiquée dans la revendication 3 pour $Y^7$, $Y^{7'}$ et respectivement $NY^7Y^{7'}$.

6. Leucocomposés de formule

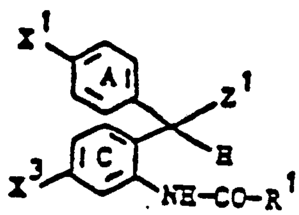

dans laquelle

$R^1$, $Z^1$, $X^1$, $X^2$, $X^3$, A, B et C ont la définition indiquée dans la revendication 1 et

lorsque $Z^1$ représente l'hydrogène et lorsque les noyaux A et C ne portent pas d'autres subsituants ou de noyaux condensés, $X^1$ et $X^3$ ne représentent pas simultanément

10 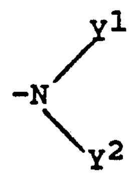 .

7. Procédé de production d'une 3,1-benzoxazine suivant la revendication 1, caractérisé en ce qu'on fait réagir un amide de formule

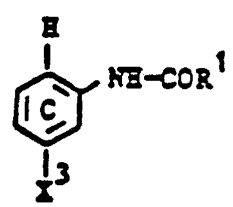

avec une cétone ou un aldéhyde de formule

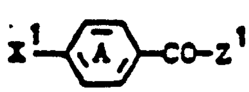

ou bien on fait réagir un composé aromatique de formule

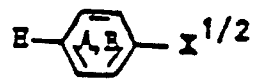

avec une cétone de formule

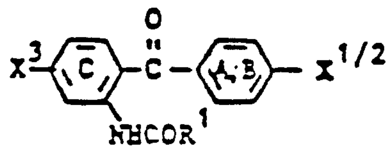

formules dans lesquelles

$R^1$, $Z^1$, $X^1$, $X^2$, $X^3$, A, B et C ont la définition indiquée dans la revendication 1.

8. Procédé de production d'une 3,1-benzoxazine suivant la revendication 1, caractérisé en ce qu'on oxyde un leucocomposé de formule

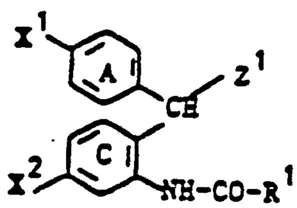

dans laquelle

$R^1$, $Z^1$, $X^1$, $X^2$, $X^3$, A, B et C ont la définition indiquée dans la revendication 1.

9. Utilisation de 3,1-benzoxazines chromogènes suivant la revendication 1 pour des supports d'enregistrement aptes au copiage par pression, thermoréactifs ou électrochromes qui contiennent un révélateur acide de couleur.

10. Support d'enregistrement sensible à la pression, thermosensible et électrosensible, caractérisé en ce qu'il contient dans son système de corps réactionnels une 3,1-benzoxazine de formule indiquée dans la revendication 1 comme substance chromogène et un révélateur acide de couleur.